Europäisches Patentamt

European Patent Office

Office européen des brevets

(19)

(11) Publication number: **0 212 532**
**A1**

# EUROPEAN PATENT APPLICATION

(12)

(21) Application number: **86111099.7**

(22) Date of filing: **11.08.86**

(51) Int. Cl.⁴: **C 12 N 15/00, C 07 K 13/00,**
**C 07 K 15/00, C 07 K 7/06,**
**G 01 N 33/53, G 01 N 33/68,**
**A 61 K 39/12, A 61 K 37/02**

(30) Priority: **12.08.85 US 765035**
**12.09.85 US 775479**

(43) Date of publication of application: **04.03.87**
**Bulletin 87/10**

(84) Designated Contracting States: **AT BE CH DE FR GB IT LI LU NL SE**

(71) Applicant: **SYNTEX (U.S.A.) INC., 3401 Hillview Avenue, Palo Alto, California 94304 (US)**
Applicant: **Martin, Malcolm A., 6408 Crane Terrace, Bethesda Maryland 20817 (US)**

(72) Inventor: **Chan, Hardy W., 2755 Saint James Road, Belmont California 94022 (US)**
Inventor: **Shelton, Earl R., 680 Lemon Street, Menlo Park California 94025 (US)**
Inventor: **Baecker, Preston A., 1644 Grosbeak, Sunnyvale California 94087 (US)**
Inventor: **Salazar, Felix H., 4151 Amaranta Avenue, Palo Alto California 94306 (US)**
Inventor: **Martin, Malcolm A., 6408 Crane Terrace, Bethesda Maryland 20817 (US)**
Inventor: **Barnett, Jim W., 220 Autumn Street, La Honda California 94020 (US)**

(74) Representative: **Barz, Peter, Dr. et al, Patentanwälte Dipl.-Ing. G. Dannenberg Dr. P. Weinhold, Dr. D. Gudel Dipl.-Ing. S. Schubert, Dr. P. Barz Siegfriedstrasse 8, D-8000 München 40 (DE)**

(54) **Method for producing fusion proteins.**

(57) Novel fusion proteins comprise an E. coli TrpLE protein segment, optionally a cleavable polypeptide linker, optionally a three-frame linker oligopeptide, and a heterologous polypeptide.

EP 0 212 532 A1

-1-

## METHOD FOR PRODUCING FUSION PROTEINS

This invention relates to a novel method for producing fusion proteins using the TrpLE gene of E. coli, to the fusion proteins so obtained and their uses, and to the modified plasmids used to obtain said proteins.

The technical and economical advantages of using recombinant DNA methodology to produce useful proteins and polypeptides from essentially any source in another organism, such as E. coli, are now well established. In general, the method comprises the construction of a self-replicating vector capable of achieving a high copy number within a host cell, in which a strong promoter-operator controls the expression of a desired heterologous gene. Subsequent transformation of a host (e.g., E. coli) with the vector, growth of the host culture to a high density and induction of the promoter

89821

25160-FF

-2-

then leads to production of the heterologous polypeptide. High yields of the desired polypeptide naturally require less purification to produce a homogeneous product, but the varied physiochemical properties of each specific polypeptide generally require an individualized purification scheme for each polypeptide.

Much emphasis has been given to the characterization of strongly inducible promoter-operator systems which allow for controlled overproduction of a heterologous polypeptide at an optimal time during the growth of the host cells. A particularly well-studied case is that of the Trp promoter-operator. See I. P. Crawford and G. V. Stauffer, Ann. Rev. Biochem., 49, 163-195 (1980). Biosynthesis of tryptophan in E. coli is catalyzed by a series of five enzymes referred to as TrpA, TrpB, TrpC, TrpD, and TrpE. The genes encoding these enzymes lie adjacent to and are controlled by the Trp promoter-operator, with the TrpE gene nearest the Trp leader. A series of deletion mutants have been isolated in which the amino terminus of the Trp leader peptide is fused to the carboxy portion of the TrpE protein so as to produce various leader-TrpE ("LE") proteins. See K. Bertrand, et al., J. Mol. Biol., 103, 319-337 (1976); G. F. Miozzari and C. Yanofsky, J. Bact., 133, 1457-1466, (1978). Because these LE proteins can be produced at levels 8-10 fold higher than the TrpE protein in the wild type configuration, these altered Trp genes have been used to produce a number of heterologous polypeptides. See D. G. Kleid, et al., GB Pat. No. 2,073,203; B. P. Nichols and C. Yanofsky, Meth. Enzymol., 101, 155-164, (1983).

The intracellular synthesis of a fusion protein by expression of a heterologous gene of interest fused to a well-expressed host gene is a favored means of achieving high levels of expression of a heterologous protein. For the production of small polypeptide hormones in E. coli, this approach has permitted both increased levels of expression and increased stability of the fusion protein compared to the unfused hormones. See K. Itakura et al., Science, 198, 1056-1063, (1977); D. Goeddel et al., Nature, 281, 544-548, (1979); J. Shine et al., Nature, 285, 456-461, (1980). It has been reported that fusion polypeptides of the TrpLE protein with either the foot-and-mouth disease virus capsid protein $VP_3$ (D. G. Kleid, et al., Science, 214, 1125-1129 (1981)) or with the influenza virus hemaglutinin protein HA (A. R. Davis et al., Gene, 21, 273-284 (1983)) are well-expressed. These fusion proteins are also extremely insoluble. Since the two viral proteins readily aggregate normally, it was thought that the aggregation of TrpLE fusion proteins was due to the physiochemical properties of the heterologous protein.

We have now discovered that, in addition to high levels of expression, the TrpLE protein can confer properties upon the fusion protein which are useful in the purification of the heterologous polypeptide. In particular, replacement of a variable portion of the LE carboxy terminal region with a heterologous polypeptide, even a small, soluble polypeptide, results in a self-aggregating fusion protein. Purification of the fusion protein is greatly simplified since the aggregates are easily enriched and concentrated in the pellet obtained by differential centrifugation of a cell lysate. This useful purification step can thereby be used with a variety of heterologous genes. Since the specific fusion site can be varied within the TrpLE gene without loss of the desired self-aggregation property, convenient restriction sites within the TrpLE gene can be used to simplify the construction of the desired plasmid vectors. This flexibility in the choice of fusion site allows other alterations in the TrpLE protein (as described herein) to enhance the purification of the heterologous polypeptide.

In addition, by using a "three-frame linker" coupled to the TrpLE gene, one may obtain a desired heterologous polypeptide regardless of its triplet reading frame (as well as the two out-of-frame "nonsense" polypeptides). Without the three-frame linker, one is forced to either select a fusion site that provides the proper reading frame orientation or to use a complicated cloning strategy, thus limiting the number of possible fusion sites. By using a three-frame linker, one may obtain the desired polypeptide easily even where the proper ("sense") reading frame is unknown.

Certain fusion proteins different from the proteins of the invention have been made using TrpLE genes in a distinctly different process. See, e.g., EPO patent applications 36,776 and 114,506.

A major advance in understanding the basis of Acquired Immunodeficiency Syndrome (AIDS) has been the isolation of a novel class of retrovirus from patients suffering from AIDS or AIDS-Related Complex (ARC). This group of viruses is variously called lymphadenopathy virus (LAV) (S. Wain-Hobson, et al., Cell, 40, 9-17 (1985)), Human T Lymphotropic Virus III (HTLV-III) (L. Ratner, et al., Nature, 313, 277-84 (1985)) and AIDS-Associated Retrovirus (ARV) (R. Sanchez-Pescador, et al., Science, 227, 484-92 (1985)). From a morphologic and biologic standpoint, this group of viruses is most closely related to the lentivirus family. So far, more than 100 isolates have been obtained by different research groups. Restriction enzyme analysis of proviral DNAs suggests a high degree of sequence polymorphism among various isolates. Comparison of nucleotide sequences between a limited number of cloned isolates suggests that sequence variability is particularly pronounced within the envelope gene.

We have sought to evaluate the significance of these structural differences among AIDS patients by comparing their individual immunological responses to viral infection. Serum samples from healthy individuals as well as ARC and AIDS patients were screened for immunoreactivity with cloned AIDS viral proteins using western blot analysis. Previous workers have used proteins derived from infectious AIDS viral particles in their assays. See, e.g., M. Sarngadharan, et al., Science, 224, 506-08 (1984); J. Schupbach, et al., Science, 224, 503-05 (1984). Our approach was to first clone and express subgenomic segments of the AIDS virus in E. coli using the TrpLE vectors described above in order to provide specific recombinant proteins or protein fragments for use as antigens in subsequent serological tests. The reagents so generated, in addition to being useful in diagnosis of viral infection, are potentially valuable as vaccine components. Other researchers have used less advantageous cloning vectors and methods to clone portions of the AIDS virus genome. See, for example, R. Gallo, et al., Science, 228, 93-96 (1985); R. Gallo, et al., Nature, 315, 151-154 (1985); F. Wong-Staal, et al., Cell, 41, 979-986 (1985).

Here we describe the construction of seven recombinant clones which span most of the AIDS viral genome. Upon induction, novel fusion proteins were produced that react immunologically with sera obtained from AIDS patients. We also disclose experiments in which the recombinant proteins were employed to screen 152 sera in order to establish which of the seven clones are most useful in detecting antibodies against the AIDS virus.

We have also discovered that fragments of certain AIDS-TrpLE fusion proteins obtained by cyanogen bromide digestion retain the immunological activity of the intact protein. By using small but immunologically active fragments of the antigenic proteins, purification problems are greatly simplified. Background reactivity is decreased and sensitivity is thereby increased.

The following terms appearing in the Specification and appended Claims are defined as follows:

The term "GRF" stands for growth hormone releasing factor. HuGRF denotes human growth hormone releasing factor, whereas PGRF and BGRF denote the porcine and bovine varieties, respectively.

The term "AIDS virus" as used herein refers to HTLV-III, ARV, LAV and related viruses, known to cause or suspected to cause AIDS, ARC, LAD, and related disorders.

The term "HBV" refers to hepatitis B virus.

The term "natural amino acid" refers to an amino acid from any source which is commonly found in nature, i.e., is a common constituent of naturally occurring proteins. Examples of natural amino acids are glycine, alanine, tryptophan, histidine, cysteine, methionine, phenylalanine, etc. An amino acid is "natural" whether it is extracted from biological sources or manufactured using chemical techniques.

The term "synthetic amino acid" refers to amino acids which are known in the art, but are not normally constituents of proteins derived from biological sources. The term "synthetic amino acids" includes, without limitation, such amino acids as norleucine, homoarginine, homoserine, γ-aminobutyric acid, and the like.

The term "sequence," as applied to a DNA sequence or a polypeptide sequence, refers to a DNA molecule or a polypeptide molecule in which the nucleotide or amino acid monomers are arranged in a particular order.

A term in the form "TrpLE·(restriction enzyme)" refers to the TrpLE deletion mutant DNA sequence from the 5' end up to the corresponding restriction enzyme recognition site. Thus, the term "TrpLE·Sst II" refers to the E. coli DNA sequence encoding the Trp promoter and the E protein up to the first Sst II restriction site. Similarly, the term "TrpLE·BssH II" refers to the E. coli DNA sequence encoding the TrpLE protein up to the first BssH II restriction site.

A term in the form "TrpLE·(restriction enzyme)-(heterologous sequence)" refers to the TrpLE deletion mutant DNA sequence encoding, from 5' to 3', the Trp promoter and the TrpLE protein up to the first corresponding restriction enzyme recognition site named, optionally followed by a three-frame linker segment, cleavable linker sequence, or both, with the heterologous DNA coding for the desired heterologous protein inserted at the restriction site of the three-frame linker (if present), at the 3' end of the cleavable linker sequence (if present), or at the 3' end of the TrpLE sequence. For example, the term "TrpLE·Sst II-HuGRF" refers to the DNA sequence which codes for the truncated LE protein, optionally a three-frame linker segment and/or a cleavable linker sequence, and human growth hormone releasing factor.

A term in the form "LE-(heterologous polypeptide)" refers to the fusion protein produced by expressing the DNA sequence TrpLE·(restriction enzyme)-(heterologous sequence). For example, LE-HuGRF$_{Leu27}$ refers to the fusion protein obtained by expressing either TrpLE·Sst II-HuGRF$_{Leu27}$ (comprising the LE polypeptide up to the Sst II site), or TrpLE·BssH II-HuGRF$_{Leu27}$ (comprising the LE polypeptide up to the BssH II site), optionally an oligopeptide encoded by a cleavable linker or a three-frame linker, and human growth releasing factor modified by replacing the amino acid at position 27 with leucine.

The term "cleavable linker sequence" refers to a nucleotide sequence which encodes for a peptide sequence which is easily cleavable by methods known in the art. For example, polypeptides containing methionine are easily cleaved using cyanogen bromide (CNBr), Asp-Pro peptide sequences are cleaved by acid hydrolysis, and Asp-Asp-Asp-Asp-Lys sequences are cleaved by the endopeptidase enterokinase. See, e.g., A. Light, et al., Anal. Biochem., 106, 199-206 (1980). To avoid denaturing the heterologous polypeptide, one should avoid using cleavable linker sequences which encode cleavable peptide sequences that are also present in the heterologous polypeptide. For example, if the heterologous protein contains internal Asp-Pro links, it is preferred to use a cleavable linker sequence that does not code for Asp-Pro. However, we have now discovered that LE-KAL-10 fusion proteins may be cleaved with CNBr, despite the presence of methionine residues within the KAL-10 polypeptide, to yield certain polypeptide fragments which exhibit the same immunoreactivity as the intact KAL-10 polypeptide.

The term "three-frame linker" or "three-frame linker sequence" refers to a set of one or more DNA sequences which, when inserted into the TrpLE coding segment of the plasmid vector, allows the subsequent insertion and expression of the heterologous sequence in any of the three reading frames. The term "three-frame linker segment" refers to the portion of the three frame linker remaining between the TrpLE gene and the heterologous sequence after the latter is inserted. In the Examples herein, 8, 10, and 12 base DNA sequences, each containing the recognition site of a particular restriction enzyme, were inserted into the TrpLE sequence. The heterologous sequence is then inserted at the enzyme recognition site in the three-frame linker, resulting in plasmids that have TrpLE-(1 base)-(restriction site)-heterologous sequence, TrpLE-(2 bases)-(restriction site)-heterologous sequence, and TrpLE-(3 bases)-(restriction site)- heterologous sequence. Thus, the heterologous sequence may be expressed from this plasmid in each of the three reading frames, one "sense" frame (which encodes the desired polypeptide) and two "nonsense" frames. After expression, the sense polypeptide is easily identified using immunological reagents.

The term "heterologous sequence" refers to a DNA molecule which encodes a protein or protein segment not native to the expression vector. Exemplary heterologous sequences encode polypeptides including, without limitation, human, bovine, or porcine growth hormone releasing factors, growth factors, bovine growth hormone, and antigenic viral or bacterial proteins, particularly AIDS viral proteins.

As applied to a cleaved dsDNA segment, the term "3' end" refers to the end of the dsDNA segment which would correspond to the 3' end of the mRNA which would be transcribed from the "sense" or coding strand.

One aspect of the invention is a bacterial expression vector for the expression of fusion proteins, comprising a TrpLE DNA gene fragment, an optional three frame linker, an optional cleavable linker, and a heterologous DNA sequence. Where both a three frame linker and a cleavable linker are used, they may appear in either order.

Another aspect of the invention is a method for producing a bacterial expression vector for the expression of fusion proteins, which method comprises providing a plasmid containing a TrpLE DNA gene, cleaving the TrpLE DNA gene with a restriction endonuclease, and inserting a heterologous DNA sequence. One may insert the optional three frame linker before or after the optional cleavable linker. The optional cleavable linker sequence may be inserted at any point in the method.

Another aspect of the invention is a fusion protein comprising a TrpLE polypeptide fragment, optionally a three frame linker segment, optionally a cleavable linker, and a heterologous polypeptide, or a fragment or derivative thereof. Where both a three frame linker and a cleavable linker are employed, they may appear in either order.

Another aspect of the invention is a method for inducing antibody formation in mammals by administering an effective amount of a fusion protein of the invention. The antibodies may be either polyclonal or monoclonal and may be used for detecting the presence of a heterologous polypeptide or for conferring immunity to viral, bacterial, or fungal infection.

Another aspect of the invention is a method for reducing or preventing viral infection in a mammal by competitive binding to cellular viral receptors by administering an effective amount of a fusion protein of the invention.

Another aspect of the invention is an immunoassay reagent for binding antibodies from mammals exposed to viruses, bacteria, or fungi, wherein said reagent is a fusion protein of the invention.

89821                                                        25160-FF

-11-

Another aspect of the invention is a vaccine for immunizing an animal against infection which vaccine comprises: a fusion protein of the invention or a portion thereof with a pharmaceutically acceptable excipient, preferably an adjuvant.

Another aspect of the invention is the AIDS virus ENV fragments KAL-A and KAL-B.

The manner in which these and other objects and advantages of the invention are obtained will become more apparent from the detailed description which follows and from the accompanying drawings, in which:

Figure 1 illustrates the construction of an intermediate vector plasmid pTrpE-#22.

Figure 2 illustrates the construction of the expression vector pTrpLE-#14.

Figure 3 illustrates the DNA and amino acid sequences predicted to encode the mRNA produced from the plasmid pTrpLE·Sst II-HuGRF$_{Leu27}$ and plasmid pTrpLE·Sst II-HuGRF$_{Leu27}$. The terminal sequences are based on information available in the literature. Amino acids of the HuGRF segment are shown in capital letters.

Figure 4 illustrates a flow chart of the purification scheme used to produce HuGRF$_{Leu27}$ from E. coli cells.

Figure 5 depicts an SDS protein gel demonstrating high production of the TrpLE, TrpLE-BssH II-HuGRF$_{Leu27}$ and TrpLE-Sst II-HuGRF$_{Leu27}$ proteins from E. coli cells.

Figure 6 depicts an SDS protein gel demonstrating the purification of TrpLE-BssH II-HuGRF$_{Leu27}$ from E. coli by cell lysis and centrifugation before CNBr cleavage.

Figure 7 depicts HPLC profiles of peptides released by CNBr treatment of TrpLE-Sst II-HuGRF$_{Leu27}$ (A) and

89821                                                    25160-FF

TrpLE-BssH II-HuGRF$_{Leu27}$ (B). The HPLC fractions in (B) were assayed for GRF activity by activation of adenylate cyclase, and the results presented in (C). The arrow in (A) indicates the fraction with peak activity.

Figure 8 depicts a western blot of TrpLE-BssH II-HuGRF$_{Leu27}$ (A) and TrpLE-Sst II-HuGRF$_{Leu27}$ (B) using anti-HuGRF antisera, after CNBr cleavage and HPLC fractionation of the fusion proteins.

Figure 9 illustrates the purity of several HuGRF$_{Leu27}$ preparations by Coomassie Brilliant Blue stain (A), and western blotting with anti-HuGRF antisera. Authentic HuGRF (S) is compared to HuGRF$_{Leu27}$ purified from two lots of TrpLE-BssH II-HuGRF$_{Leu27}$ (lanes 1, 2) and TrpLE-Sst II-HuGRF$_{Leu27}$ (lane 3). Unmarked lanes contain molecular weight standards.

Figure 10 depicts the HPLC profiles of lots 1, 2, and 3 described in Figure 9.

Figure 11 is a map of the AIDS virus genome illustrating the locations of the specific clones described in relation to the major open reading frames of the virus (GAG, POL, ENV, A, and B).

Figure 12 illustrates a reference pattern of TrpLE-AIDS fusion proteins and AIDS virus infected cells (BAG) visualized by western blotting with a pool of sera from three AIDS patients.

Figure 13 illustrates results from the western blotting analysis of 19 human serum samples. Each serum sample was used to probe the various TrpLE-AIDS fusion proteins (GAG, POL, ENV), the TrpLE vector without an insert (VECTOR), and AIDS virus infected cells (AIDS). The numbers above each panel indicate the number of positively reacting serum samples.

Figure 14 illustrates Western blot results comparing the immunoreactivity of CNBr treated LE-GAG fusion proteins generated by pSB8-Gag-#25 with LE-ENV fusion proteins produced by pTrpLE-KAL-ENV-#10.

Figure 15A shows an SDS-polyacrylamide gel stained with Coomasie Blue stain showing the proteins produced by pSB12-854ΔHB. Figure 15B depicts western blot analysis of the same E. coli extracts of pSB-854ΔHB demonstrating that the 35 Kd protein is the only immunoreactive protein entity.

Figures 16A and 16B depict the HPLC fractionation and western blot analysis of the CNBr-cleaved fragments obtained in Example 16.

One aspect of the invention is a bacterial expression vector for the expression of fusion proteins, which vector comprises a TrpLE DNA gene fragment and a heterologous DNA sequence. A preferred subgenus is the vector which further comprises a three-frame linker segment between said TrpLE fragment and said heterologous DNA sequence. A preferred class of the invention is the vector which further comprises a cleavable linker sequence between said TrpLE fragment and said heterologous DNA sequence, especially where said cleavable linker encodes Met, Asp-Pro, or Asp-Asp-Asp-Asp-Lys. A preferred subclass is the vector wherein said heterologous DNA sequence encodes: human, bovine, or porcine growth hormone releasing factor or a biologically active derivative or fragment thereof; an antigenic viral protein or a biologically active derivative or fragment thereof; or a growth factor or hormone or a biologically active derivative or fragment thereof. A preferred species is the vector encoding an antigenic viral protein in which the antigenic viral protein is an AIDS protein, particularly an ENV polypeptide, and especially the polypeptide KAL-10.

Another preferred species is that wherein the antigenic viral protein is a pre-S sequence of the surface antigen of HBV (hepatitis B virus). Another preferred species is the vector encoding a growth hormone releasing factor derivative, especially where the derivative is HuGRF$_{Leu27}$, BGRF$_{Leu27}$, or PGRF$_{Leu27}$. Another preferred species is the vector encoding a growth hormone, especially where the growth hormone is bovine growth hormone. Presently preferred embodiments of the invention are the plasmids pTrpLE-KAL, pTrpLE-KAL-ENV-#10, pSB12-854, pSB12-854ΔHB, pTrpLE·Sst II-HuGRF$_{Leu27}$, pTrpLE·BssH II-HuGRF$_{Leu27}$-#5, pTrpLE·Sac II-PGRF, and pTrpLE BssH II-BGRF$_{Leu27}$#15, as defined in the examples.

Another aspect of the invention is a method for producing a bacterial expression vector for the expression of fusion proteins, which method comprises:

providing a plasmid containing a TrpLE DNA gene;

cleaving the TrpLE DNA gene with a restriction endonuclease; and

inserting a heterologous DNA sequence. A preferred subgenus is the method which further comprises inserting a cleavable linker sequence before or after inserting the heterologous sequence. A preferred class of the invention is the method wherein the cleavable linker sequence encodes Met, Asp-Pro, or Asp-Asp-Asp-Asp-Lys, especially Met or Asp-Pro. A preferred subclass is the method wherein said heterologous DNA sequence encodes: human, bovine, or porcine growth hormone releasing factor or a biologically active derivative or fragment thereof; an antigenic viral protein or a biologically active derivative or fragment thereof; or a growth factor or hormone or a biologically active derivative or fragment thereof. A preferred species is the method wherein the heterologous sequence encodes an antigenic viral protein in which the antigenic viral protein is an AIDS protein, particularly an ENV polypeptide, and especially the polypeptide KAL-10. Another preferred species is that wherein the antigenic viral protein is a pre-S sequence of HBV (hepatitis B virus). Another preferred species is the method wherein the heterologous sequence encodes a growth hormone releasing factor derivative, especially where the derivative is $HuGRF_{Leu27}$, $BGRF_{Leu27}$, or $PGRF_{Leu27}$. Another preferred species is the method wherein the heterologous sequence encodes a growth hormone, especially where the growth hormone is bovine growth hormone.

Another preferred subgenus is the method for producing a bacterial expression vector for the expression of fusion proteins, which method comprises:

selecting a plasmid containing a TrpLE DNA gene;

cleaving the TrpLE DNA gene with a restriction endonuclease;

inserting a three frame linker; and

inserting a heterologous DNA sequence within the three frame linker. A preferred subclass is the method wherein said heterologous DNA sequence encodes: human, bovine, or porcine growth hormone releasing factor or a biologically active derivative or fragment thereof; an antigenic viral protein or a biologically active derivative or fragment thereof; or a growth factor or hormone or a biologically active derivative or fragment thereof. A preferred species is the method wherein the heterologous sequence encodes an antigenic viral protein in which the antigenic viral protein is an AIDS protein, particularly an ENV polypeptide, and especially the polypeptide KAL-10. Another preferred species is that wherein the antigenic viral protein is a pre-S sequence of HBV (hepatitis B virus). Another preferred species is the method wherein the heterologous sequence encodes a growth hormone releasing factor derivative, especially where the derivative is $HuGRF_{Leu27}$, $BGRF_{Leu27}$, or $PGRF_{Leu27}$. Another preferred species is the method wherein the heterologous sequence encodes a growth hormone, especially where the growth hormone is bovine growth hormone.

Another aspect of the invention is a fusion protein comprising a TrpLE polypeptide fragment and a heterologous polypeptide, or a fragment or derivative thereof. A preferred subgenus of the invention is the fusion protein which further comprises a three-frame linker oligopeptide segment between the TrpLE polypeptide fragment and the heterologous polypeptide. A preferred subclass is the fusion protein wherein the heterologous polypeptide is: human, bovine, or porcine growth hormone releasing factor or a biologically active derivative or fragment thereof; an antigenic viral protein or a biologically active derivative or fragment thereof; or a growth factor or hormone or a biologically active derivative or fragment thereof. A preferred species is that wherein the antigenic viral protein is a pre-S sequence of HBV (hepatitis B virus). Another preferred species is the fusion protein wherein the heterologous polypeptide is a growth hormone releasing factor derivative, especially where the derivative is $HuGRF_{Leu27}$, $BGRF_{Leu27}$, or $PGRF_{Leu27}$. Another preferred species is the fusion protein wherein the heterologous polypeptide is a growth hormone, especially where the growth hormone is bovine growth hormone. Another preferred species is the fusion protein wherein the heterologous polypeptide is an antigenic viral protein in which the antigenic viral protein is an AIDS protein, particularly an ENV polypeptide, and especially the polypeptide KAL-10. A particularly preferred species is an immunoreactive KAL-10 fragment, especially KAL-A, KAL-B, or a mixture of the two, where KAL-A and KAL-B have the following sequences:

Kal-A:
```
Met Arg Asp Asn Trp Arg Ser Glu Leu Tyr Lys
Tyr Lys Val Val Lys Ile Glu Pro Leu Gly Val
Ala Pro Thr Lys Ala Lys Arg Arg Val Val Gln
Arg Glu Lys Arg Ala Val Gly Ile Gly Ala Leu
Phe Leu Gly Phe Leu Gly Ala Ala Gly Ser Thr X
```

Kal-B:
```
Met Thr Leu Thr Val Gln Ala Arg Gln Leu Leu
Ser Gly Ile Val Gln Gln Gln Asn Asn Leu Leu
Arg Ala Ile Glu Ala Gln Gln His Leu Leu Gln
Leu Thr Val Trp Gly Ile Lys Gln Leu Gln Ala
Arg Ile Leu Ala Val Glu Arg Tyr Leu Lys Asp
Gln Gln Leu Leu Gly Ile Trp Gly Cys Ser Gly
Lys Leu Ile Cys Thr Thr Ala Val Pro Trp Asn
Ala Ser Trp Ser Asn Lys Ser Leu Glu Gln Ile
Trp Asn Asn X
```

wherein X represents homoserine lactone or its hydrolysis or amidation products, methionine, or another naturally occurring amino acid. Other subsets and analogs of the two sequences set forth above have immunological activity and are within the scope of the invention. Subsets are generated from the above sequences by deleting amino acyl residues from either or both ends of the sequence, such that the resulting polypeptide retains at least substantially the same immunoreactivity. Analogs are generated by replacing up to three amino acids with other natural or synthetic amino acids known to those skilled in the art by recombinant or chemical methods, such that the resulting polypeptide retains at least substantially the same immunoreactivity. Analogs include subsets and sequences which have up to three amino acyl residues within the sequence deleted.

Another preferred subgenus of the invention is the fusion protein which further comprises a cleavable linker oligopeptide between the TrpLE polypeptide fragment and the heterologous polypeptide. A preferred class of the invention is the method wherein the cleavable linker oligopeptide is Met, Asp-Pro, or Asp-Asp-Asp-Asp-Lys, especially Met or Asp-Pro. A preferred subclass is the fusion protein wherein the heterologous polypeptide is: human, bovine, or porcine growth hormone releasing factor or a biologically active derivative or fragment thereof; an antigenic viral protein or a biologically active derivative or fragment thereof; or a growth factor or hormone or a biologically active derivative or fragment thereof. A preferred species is the fusion protein wherein the heterologous polypeptide is an antigenic viral protein in which the antigenic viral protein is an AIDS protein, particularly an ENV polypeptide, and especially the polypeptide KAL-10. Another preferred species is that wherein the antigenic viral protein is a pre-S sequence of HBV (hepatitis B virus). Another preferred species is the fusion protein wherein the heterologous polypeptide is a growth hormone releasing factor derivative, especially where the derivative is $HuGRF_{Leu27}$, $BGRF_{Leu27}$, or $PGRF_{Leu27}$. Another preferred species is the fusion protein wherein the heterologous polypeptide is a growth hormone, especially where the growth hormone is bovine growth hormone.

Another aspect of the invention is a method for inducing antibody formation in mammals by administering an effective amount of a fusion protein or the invention. A preferred subgenus is the method wherein said antibodies are polyclonal or monoclonal and are useful for detecting the presence of a heterologous polypeptide. A preferred class of the invention is the method in which the heterologous polypeptides to be detected are viruses. A preferred species is that wherein the virus is AIDS virus or HBV. Another preferred subgenus is the method wherein said antibodies are neutralizing antibodies useful for conferring immunity to viral, bacterial, or fungal infection. A preferred species is that in which the antibodies confer immunity to AIDS. Another preferred species is that in which the antibodies confer immunity to HBV.

Another aspect of the invention is a method for reducing or preventing viral infection in a mammal by competitive binding to cellular viral receptors by administering an effective amount of a fusion protein, wherein said fusion protein comprises a TrpLE polypeptide fragment and a virus polypeptide recognized by a mammalian cell virus receptor. A preferred species is the method in which the virus polypeptide is an AIDS virus polypeptide.

Another aspect of the invention is an immunoassay reagent for binding antibodies from mammals exposed to viruses, bacteria, or fungi, wherein said reagent is a fusion protein comprising a TrpLE polypeptide fragment and an antigenic viral, bacterial or fungal polypeptide. A preferred class is the immunoassay reagent wherein said polypeptide is a viral polypeptide. A preferred subclass is the reagent wherein the viral polypeptide is an ENV or surface protein or a fragment thereof. A preferred species is the reagent wherein the ENV protein is an AIDS protein fragment, especially KAL-10. A particularly preferred species is an immunoreactive KAL-10 fragment, especially KAL-A, KAL-B, or a mixture of the two, or a subset or analog of KAL-A or KAL-B. Another preferred species is the reagent wherein the surface protein is the pre-S sequence of the surface antigen of HBV.

TrpLE

The fusion between the DNA sequences encoding the LE protein and the heterologous polypeptide is made in a manner such that various distal portions of the LE protein will be replaced, in the same translational reading frame, by the heterologous polypeptide. Additionally, other amino acid insertions, deletions and substitutions in the LE protein may be introduced so as to facilitate the purification of the desired product.

Cells are transformed by addition of the expression vehicles of the invention and grown under conditions in which the exogenous promoter-operator is repressed. For instance, in the case of the E. coli tryptophan promoter-operator (Trp promoter), cells are grown in the presence of added tryptophan. This represses the Trp promoter such that cell growth can proceed normally, without deleterious effect due to overexpression of the fusion protein. When the recombinant culture reaches a cell density appropriate for industrial production of the polypeptide, the external source of repression (e.g., tryptophan) is removed, or, alternatively, an external inducer (e.g., indole acrylic acid) is added. This derepresses the exogenous promoter-operator (e.g., Trp promoter) and highly efficient expression of the heterologous insert occurs.

The invention provides a variety of useful intermediates and end products, including heterologous polypeptide-containing fusion proteins which can be easily separated from the remainder of the bacterial proteins by virtue of the self-aggregating properties conferred by the LE protein portion. The fusion protein may then be specifically cleaved to release the desired heterologous polypeptide, whose purification is facilitated by the choice of the fusion site within the LE protein. Alternatively, one may use the fusion protein without cleavage, e.g., to produce vaccines or monoclonal antibodies for immunoassays.

The original isolation of the TrpLE protein cloning region was the result of genetic manipulations of the E. coli tryptophan promoter-operator. See K. Bertrand, C. Squires and C. Yanofsky, J. Mol. Biol., 103, 319-337 (1976); G. F. Miozzari and C. Yanofsky, J. Bact., 133, 1457-1466 (1978). One of these original deletions, TrpLE1413, is available on plasmid pVV1 (B. P. Nichols and C. Yanofsky, Meth. Enzymol., 101, 155-164 (1983)) and was used to construct the LE protein vectors employed in the examples herein. The essential features of a DNA deletion required to form an effective LE protein vector for the purposes of this invention are, relative to the Trp promoter-operator (C. Yanofsky et al., Nuc. Acids Res., 9, 6647-6668 (1981)):

a 5' terminal breakpoint near a base coding for amino acid #10 of the TrpL peptide;

a 3' terminal breakpoint about two-thirds of the way through the TrpE coding region, preferably near amino acid #339.

Such a deletion removes the Trp promoter-operator attenuator region, allowing for higher level of gene expression, and preserves a region of the TrpE protein from about amino acid #339 to the carboxy terminal amino acid, which is preferred for its ability to self-aggregate even when heterologous proteins are substituted for a portion of the carboxy coding region.

A preferred embodiment of the invention is the LE vector plasmid pTrpLE-#14. This is a pBR322-based plasmid containing the E. coli Trp promoter-operator and LE coding region of 191 amino acids from pVV1, followed in turn by a portion of the TrpD gene and a transcription terminator fragment from the E. coli RNA polymerase-β subunit gene (RpoC: See C. Squires et al., Nucleic Acids Res., 9, 6827-6840 (1981)). The terminator provides for termination of mRNA synthesis, thereby preventing transcription beyond the gene of interest which could interfere with plasmid replication or stability. Transcripts of the terminator region may also form structures which stabilize the 3' end of the mRNA against degradation.

The expression of the LE protein either alone or fused to a heterologous gene is conveniently brought about by the tryptophan promoter in plasmid pTrpLE-#14 and its derivatives as described herein. It is appreciated, however, that any similar well-characterized promoter-operator DNA region known to be capable of regulating gene expression in E. coli could be substituted for the Trp promoter used in these particular examples. The promoter region of the Trp promoter-operator commends itself for this use because it is so well-characterized (I. P. Crawford and G. V. Stauffer, Ann. Rev. Biochem., 49, 163-195 (1980)) and can be easily induced to produce high levels of mRNA and hence protein products by growth in media either lacking tryptophan or containing the inducer compound indole acrylic acid (IAA). Other suitable control elements might include the following, or selected regions thereof; the Lac promoter, the Tac promoter, the Arabinose, Galactose or Alkaline Phosphatase operons, or a promoter-operator from bacteriophage lambda.

Likewise, the provision of a transcription termination function by the RpoC DNA fragment and the selectable marker of ampicillin resistance employed in pTrpLE-#14 were used by way of an example and are not meant to limit the scope of this invention.

GRF

Growth hormone releasing factor (GRF) is a small (44 amino acid) polypeptide secreted by the hypothalamus which stimulates the release of growth hormone by the pituitary in mammals. GRF is useful for increasing weight gain in animals, and for increasing milk production in lactating mammals. GRFs from different species typically vary by a few amino acids in composition, as illustrated below:

|          | 1   |     |     |     | 5   |     |     |     |     | 10  |
|----------|-----|-----|-----|-----|-----|-----|-----|-----|-----|-----|
| HuGRF :  | Tyr | Ala | Asp | Ala | Ile | Phe | Thr | Asn | Ser | Tyr |
| PGRF :   | Tyr | Ala | Asp | Ala | Ile | Phe | Thr | Asn | Ser | Tyr |
| BGRF :   | Tyr | Ala | Asp | Ala | Ile | Phe | Thr | Asn | Ser | Tyr |

|     |     |     |     | 15  |     |     |     |     | 20  |
|-----|-----|-----|-----|-----|-----|-----|-----|-----|-----|
| Arg | Lys | Val | Leu | Gly | Gln | Leu | Ser | Ala | Arg |
| Arg | Lys | Val | Leu | Gly | Gln | Leu | Ser | Ala | Arg |
| Arg | Lys | Val | Leu | Gly | Gln | Leu | Ser | Ala | Arg |

|     |     |     |     | 25  |     | 27  |     |     | 30  |
|-----|-----|-----|-----|-----|-----|-----|-----|-----|-----|
| Lys | Leu | Leu | Gln | Asp | Ile | Met | Ser | Arg | Gln |
| Lys | Leu | Leu | Gln | Asp | Ile | Met | Ser | Arg | Gln |
| Lys | Leu | Leu | Gln | Asp | Ile | Met | Asn | Arg | Gln |

|     |     |     |     | 35  |     |     |     |     | 40  |
|-----|-----|-----|-----|-----|-----|-----|-----|-----|-----|
| Gln | Gly | Glu | Ser | Asn | Gln | Glu | Arg | Gly | Ala |
| Gln | Gly | Glu | Arg | Asn | Gln | Glu | Gln | Gly | Ala |
| Gln | Gly | Glu | Arg | Asn | Gln | Glu | Gln | Gly | Ala |

|     |     |     | 44       |
|-----|-----|-----|----------|
| Arg | Ala | Arg | Leu-NH$_2$ |
| Arg | Val | Arg | Leu-NH$_2$ |
| Lys | Val | Arg | Leu-NH$_2$ |

Production in E. coli of human growth hormone releasing factor (HuGRF) was achieved by fusing the HuGRF DNA sequence to the TrpLE sequence. In a preferred embodiment, the fusion between the HuGRF and the LE protein coding regions was via the codon for methionine (ATG), to allow for release of the HuGRF via specific cleavage at Met using CNBr. To prevent cleavage at the methionine in position 27 of HuGRF, position 27 was changed to a leucine in the synthetic DNA which was

89821                                                      25160-FF

cloned into the LE protein vector. Leucine was chosen since it is most often substituted for methionine in naturally occurring mutations, but other naturally occurring amino acids may be substituted to prevent CNBr cleavage. The synthetic DNA was also designed to maximize the use of amino acid codons which are favored in highly expressed $\underline{E}$. $\underline{coli}$ proteins (R. Grantham et al., Nucleic Acids Res., $\underline{9}$, r43-r74 (1981)), to minimize potential secondary structure in the resultant mRNA, and to provide convenient restriction sites which are useful for screening the desired clone and facilitating future alternatives in the DNA sequence. The synthetic DNA coding for the HuGRF$_{Leu27}$ was ligated to either the Sac II site of the LE gene, replacing the carboxy terminal 73 amino acids of the LE protein, or to the BssH II site, replacing 11 amino acids.

Although the examples cited interpose a methionine between the LE protein and the heterologous polypeptide, it will be appreciated that this junction may comprise any combination of the twenty naturally occurring amino acids, or none at all, and need not be specifically cleavable by chemical or enzymatic means, yet still remain within the scope of the invention.

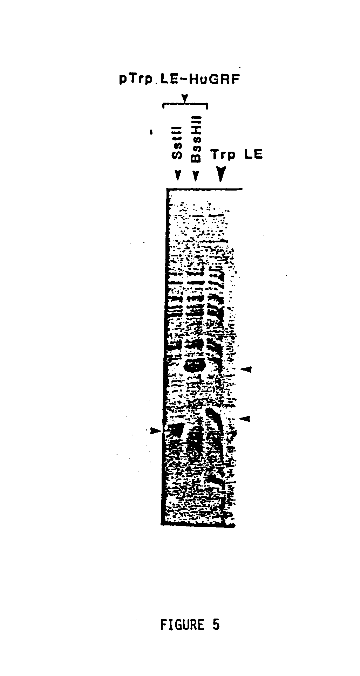

Good induction of expression from the Trp promoter-operator of plasmids encoding the HuGRF$_{Leu27}$ fused to either the BssH II or Sac II sites of the LE gene was readily achieved (Figure 5). The kinetics of synthesis and the extent of accumulation of the HuGRF$_{Leu27}$ were dependent upon the composition of the culturing media. Indole acrylic acid (IAA) was required to maximally induce synthesis in a relatively rich medium, L broth. In contrast, inclusion of IAA in a minimal medium, M-9, altered only the kinetics of accumulation, but not the final level, of the LE-HuGRF$_{Leu27}$. As much as 35% of total protein was LE-HuGRF$_{Leu27}$ after growth in M-9, compared to 15-20% in L broth plus IAA. Maximal stability of the plasmids is obtained by growth in media containing 20-100 µg/ml tryptophan until expression is desired.

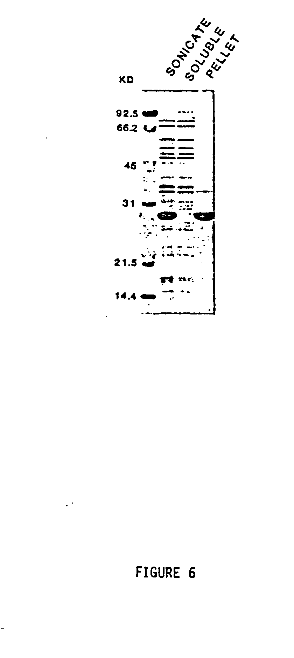

Purification of the HuGRF$_{Leu27}$ was commenced by recovery of the insoluble aggregate of LE-HuGRF$_{Leu27}$ protein from the lysed cells by low speed centrifugation (Figure 6). This material was treated with CNBr in 70% formic acid. Formic acid serves as a preferred solvent to dissolve the LE fusion protein aggregates in the pellet and thereby provide a homogenous solution for further processing. Where the fusion protein itself is the desired product, solvents such as SDS or urea may be used to dissolve the LE fusion protein aggregates prior to further processing. After removal of excess CNBr and formic acid, the HuGRF$_{Leu27}$ peptide was extracted from the insoluble material with 1N acetic acid. The extract was then dialyzed into dilute buffer before fractionation by reverse phase HPLC.

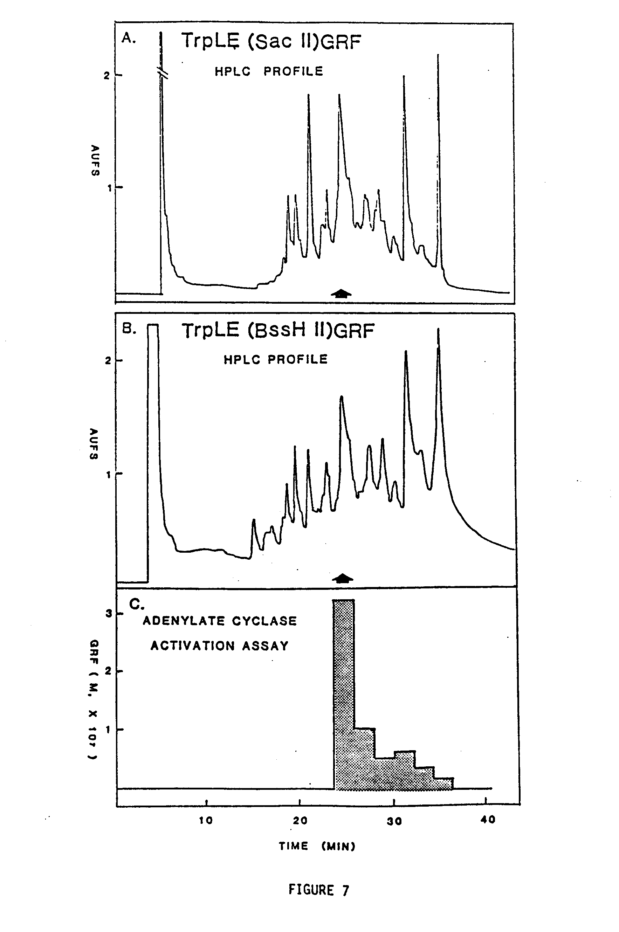

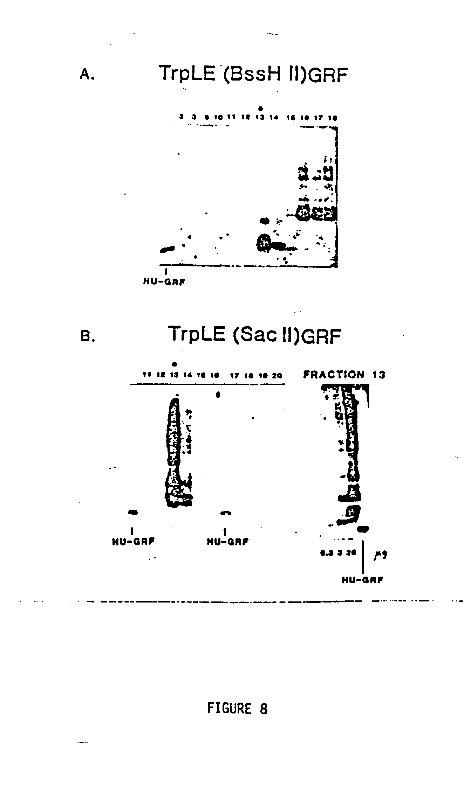

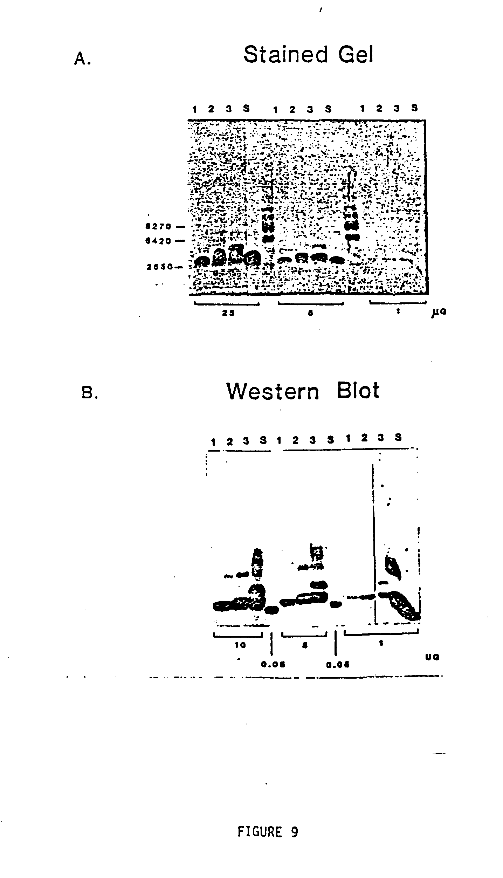

Good resolution of HuGRF$_{Leu27}$ has been achieved using either reverse phase HPLC or a combination of CM-cellulose and HPLC (Figures 7 and 8). The latter approach was preferred for material isolated from the TrpLE·Sac II-HuGRF$_{Leu27}$ clone since CM-cellulose removed some contaminants which were not possible to separate by HPLC alone. Fractionation of the dialysate prepared from the TrpLE·BssH II-HuGRF$_{Leu27}$ clone (Fig. 7B) or the TrpLE·Sac II-HuGRF$_{Leu27}$ clone (Fig. 7A) on HPLC gave HPLC profiles of total protein that were nearly identical. In contrast, when analyzed by the sensitive western blotting technique (Fig. 8B), the HuGRF$_{Leu27}$ from the Sac II clone was shown to elute from the HPLC with immunoreactive material of higher molecular weight. When the dialysate from the Sac II cloned HuGRF$_{Leu27}$ was first fractionated on CM-cellulose, the HPLC-purified material was substantially free of these contaminants (Figure 9B). $HuGRF_{Leu27}$ of the greatest purity was isolated from the BssH II clone, even without a CM-cellulose step, because the immunoreactive contaminants were well separated by HPLC (Fig. 8A). The identity of these immunoreactive products was based upon their co-migration with the immunoreactive products seen with limiting amounts of CNBr, their ability to stimulate adenylate cyclase in an in vitro GRF assay, and their reactivity with anti-HuGRF sera.

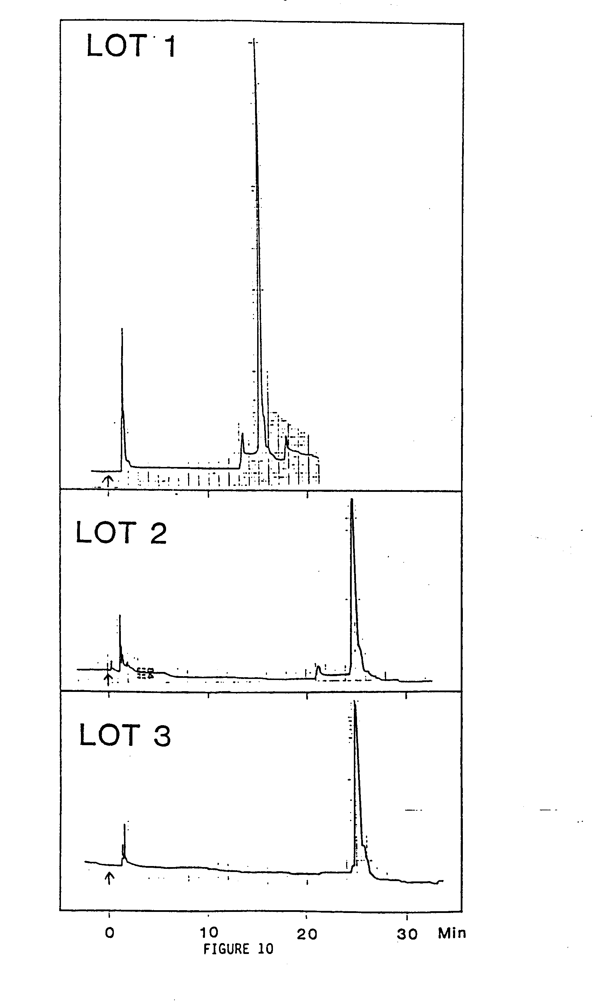

The amino acid sequence of the material isolated by preparative HPLC was found to be correct, including the substitution of Leu for Met at position 27. Samples with a purity of greater than 95% (Figures 9 and 10) were shown to stimulate both adenylate cyclase in an in vitro assay using bovine pituitary cell extracts and growth hormone release from pituitary cells in culture.

The porcine analog of HuGRF (PGRF) as isolated from pig hypothalami differs in amino acid sequence from HuGRF at three positions towards the carboxy terminus: Ser-34 → Arg; Arg-38 → Gln; Ala-42 → Val (Bohlen et al., Biochem. Biophys. Res. Comm., 116, 726-734 (1983)). Expression of the $PGRF_{Leu27}$ analog of PGRF as a fusion to the TrpLE protein was therefore achieved by specifically deleting the later portion of the LE·BssH II-$HuGRF_{Leu27}$ coding region using restriction endonucleases, and substituting in its place a synthetic DNA fragment coding for the carboxy terminus of PGRF. As in the case of the $HuGRF_{Leu27}$ hormone, the LE·BssH II-$PGRF_{Leu27}$ fusion protein was produced in high yield from induced cells, quantitatively sedimented, and $PGRF_{Leu27}$ easily purified by the identical procedure used for the $HuGRF_{Leu27}$. Material isolated by preparative HPLC was shown to be pure by analysis of the amino acid composition and biologically active by the same criteria applied to HuGRF$_{Leu27}$ above.

The bovine analog of HuGRF (BGRF), as isolated from bovine hypothalami differs from the HuGRF in five positions towards the carboxy terminus: Ser-28 → Asn; Ser-34 → Arg; Arg-38 → Gln; Arg-41 → Lys; Ala-42 → Val. In an analogous manner to the construction and expression of the PGRF$_{Leu27}$, a clone encoding the LE·BssH II-BGRF$_{Leu27}$ polypeptide was constructed, expressed and the BGRF$_{Leu27}$ successfully purified and shown to be pure and biologically active.

The same procedure was thereby successfully applied for expression of the LE·BssH II-PGRF$_{Leu27}$ fusion polypeptide and purification of the GRF$_{Leu27}$ from three different sources, human, porcine and bovine.

AIDS

We have described above the construction and utilization of an E. coli expression vector to produce, in high yield, a number of animal-derived growth hormone releasing factors. This vector, designated pTrpLE, uses the strong bacterial promoter from the tryptophan promoter-operator to express a novel protein known as LE. The expression of each growth hormone releasing factor was achieved by fusion of the respective growth hormone releasing factor gene, in the proper translational reading frame, to the carboxy end of the LE gene. The LE carrier protein is itself a fusion protein between the amino terminus of the TrpE protein and a segment of the Trp leader. By linking foreign gene sequences in frame and downstream from the LE protein, we have successfully employed this and related vectors to express a number of heterologous polypeptides in high yield.

By inserting synthetic BamH I linkers of various lengths (e.g., 8, 10 and 12 nucleotides) at the unique BssH II site of pTrpLE, we have created a new set of expression vectors which allow expression of any translational open reading frame that is flanked with compatible restriction enzyme ends. For instance, bacterial or viral DNA can be cleaved (completely or partially) with Sau3A (GATC) and ligated to the three vectors, which have been previously cleaved with BamH I (GGATCC) and treated with alkaline phosphatase. Using antibodies directed against the LE carrier or the heterologous peptide, the transformants producing recombinant immunoreactive proteins can be identified.

To generate an expression library for the AIDS viral genome, a fragment of DNA derived from pBenn#20 was partially cleaved with restriction endonuclease Sau3A and inserted into the three open-reading-frame vectors, pSB8, pSB10 and pSB12. Plasmid pBenn#20 had been previously constructed as a recombinant plasmid containing an 8 Kb segment of the proviral DNA, as well as 9 Kb of flanking cellular DNA.

Transformants were screened for the presence of AIDS viral inserts by colony hybridization with radioactively labelled DNA fragments derived from pBenn#20. The results indicated that greater than 80% of the transformants contained inserts. Moreover, the transformants could thereby be divided into subgroups based on the region of the AIDS viral genome from which the inserts were derived. Selected clones from these subgroups were then tested for their ability to produce protein molecules that were immunoreactive with anti-AIDS virus sera. Protein extracts prepared from induced recombinant bacterial cultures were subjected to western blot analyses using pooled sera from three patients, each of whom had previously been identified as producing anti-AIDS virus antibodies.

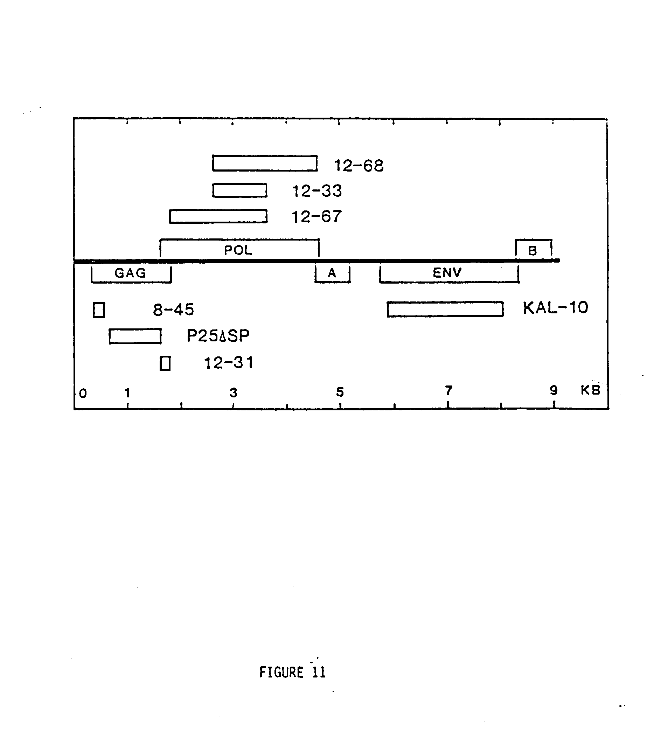

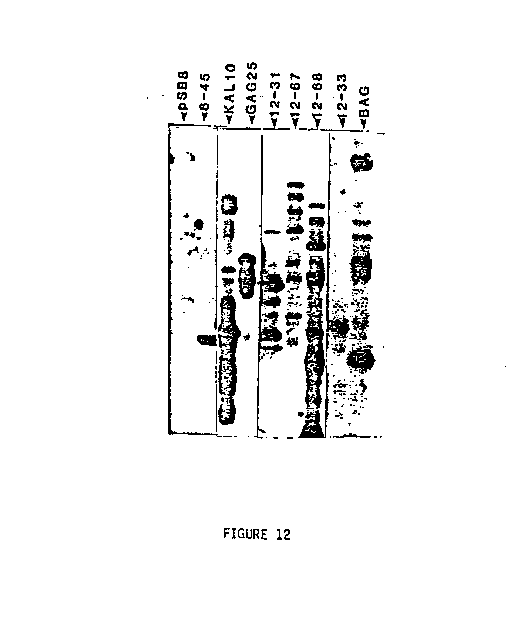

Plasmid DNAs were then prepared from positively reactive colonies and their nucleotide sequences determined. The location of each insert within the AIDS provirus was established by comparing the DNA sequence at the boundaries of the insert with the published nucleotide sequence of the AIDS virus genome (Table 1). The results confirmed that each antibody-reactive clone contained an AIDS viral DNA fragment inserted in the proper orientation and correct reading frame with regard to the transcription and translation of the LE carrier protein. Figure 11 summarizes the genomic locations of five of the Sau3A recombinant clones, and Figure 12 shows their protein profiles as revealed by western blot analysis.

In addition to the genomic library derived from the AIDS virus, which was based on the insertion of random Sau3A fragments into the three open-reading-frame vectors, two additional clones containing specific AIDS virus DNA fragments were constructed. (Figure 11)

The first specific construct utilized an LE-derived vector (designated pTrpLE-KAL) in which the nucleotide sequence distal to the unique BssH II site was modified to contain cleavage sites for both Kpn I and BamH I, as well as to encode the pentapeptide Asp-Asp-Asp-Asp-Lys. This peptidyl linkage can serve as a specific recognition site for cleavage by the endopeptidase enterokinase.

Into pTrpLE-KAL was inserted a 2.15 Kb Kpn I to BamH I DNA fragment of AIDS virus DNA from plasmid pBenn#20. This fragment spans a major portion of the ENV gene. Upon induction the resultant plasmid, known as pTrpLE-KAL-ENV-#10, produced a reproducible pattern of protein products on SDS gels that were strongly immunoreactive on western blots incubated with the

0212532

-30-

anti-AIDS sera (Figure 12). This demonstrated that the AIDS viral gene was inserted in the correct translational reading frame and downstream from the LE gene.

A second specific clone, designated pSB-Gag-#25, was constructed with a 0.95 Kb Pvu II to Bgl II DNA fragment from the AIDS virus GAG gene (Figure 11) inserted in the correct reading frame downstream from the LE gene. Upon induction, this transformant produced multiple polypeptides which were immuno-reactive with anti-AIDS sera (Figure 12).

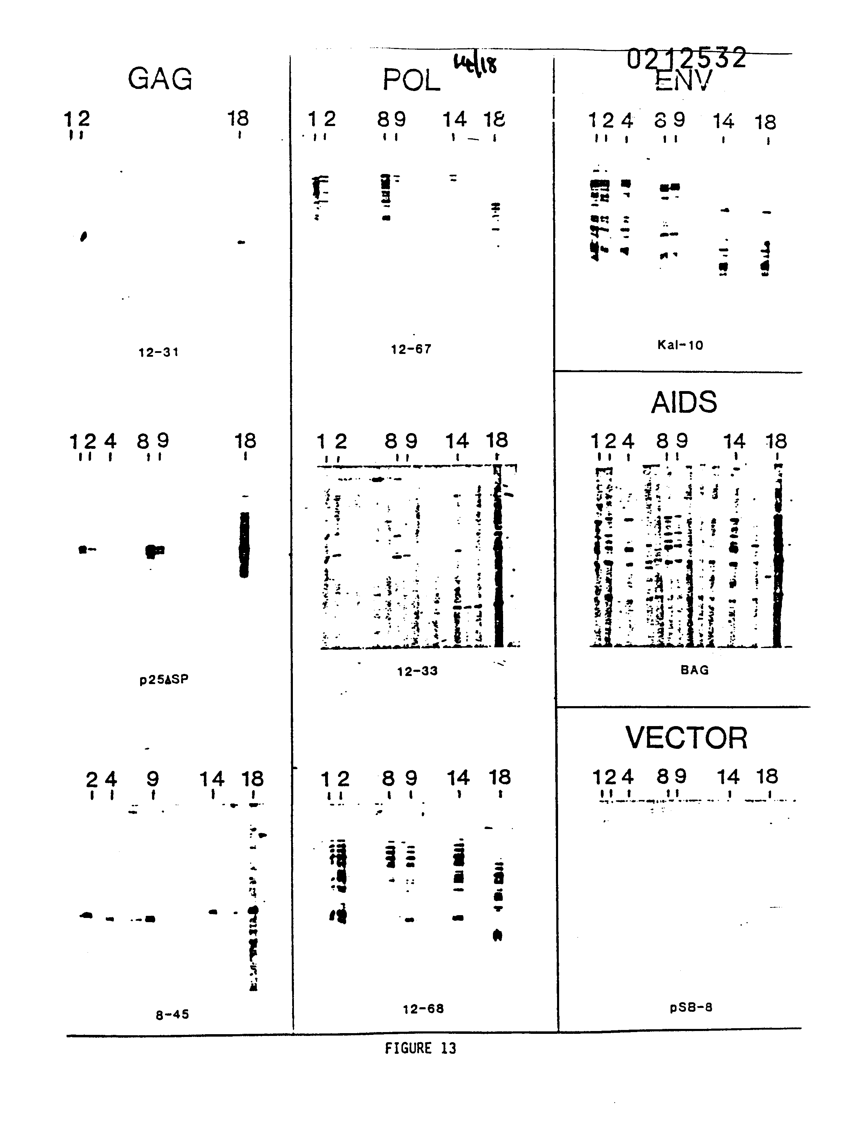

Western blotting was used with sera from patients suffering from AIDS or related diseases to identify viral target antigens. The expression vectors described herein were used to produce the AIDS proteins which were used as antigens in the western blot. An envelope-derived protein was most clearly and consistently recognized by antibodies from patients with lymphadenopathy, ARC, and AIDS (Figure 13). However, a preponderance of the members of high risk groups (e.g., homosexuals and intravenous drug users) also carried antibodies to the ENV protein.

While antibodies to GAG and POL proteins have been reported to be useful indicators of AIDS viral infection, our results demonstrate that only a subset of the individuals with antibodies to ENV also have antibodies to GAG or POL proteins. Conversely, we identified no individuals with antibodies to GAG or POL who did not also have antibodies to ENV. Thus, the recombinant AIDS ENV proteins, produced by the ENV clone pTrpLE-KAL-ENV-#10, are more appropriate diagnostic reagents for AIDS virus infection than proteins derived from other regions of the viral genome, such as GAG or POL. The multiple ENV related protein species produced by pTrpLE-KAL-ENV-#10 provide a reproducible recognition pattern on western blots to facilitate unequivocal

89821                                                   25160-FF

interpretation of immunoassay results. Production of these ENV related protein reagents in E. coli is safer and more economical than production using AIDS-infected animal cell cultures.

The origin and function of the sequence variability in the AIDS proviral DNAs, especially prevalent in the ENV gene, is not yet known. The virus cloned and sequenced herein was originally obtained as an LAV virus stock from the group at the Institut Pasteur. Although the bacterially produced LE-ENV protein lacks the glycosylation normally present and encompasses only a portion of the entire ENV open reading frame, antibodies from all AIDS and ARCS patients reacted strongly with the LE-ENV fusion protein. Thus, the sequence variability of AIDS proviral DNA does not seem to present a problem in diagnosis using portions of the ENV open reading frame. The AIDS ENV polypeptide is thus useful as a diagnostic reagent.

It has now been found that certain fragments of LE-ENV fusion proteins retain the immunoreactivity exhibited by the intact fusion protein or intact ENV protein. Upon comparision between several AIDS strains, it was found that these fragments display high (>90%) sequence conservation. In other words, these fragments contain sequences which are identical or nearly identical regardless of which strain the sequence was derived from. Two especially preferred fragments, a 55 amino acid section (Kal-A) and a 91 amino acid section (Kal-B), can be obtained by CNBr digestion of KAL-IO. These fragments have the following sequences:

Kal-A:

```
Y   Asp Asn Trp Arg Ser Glu Leu Tyr Lys Tyr
Lys Val Val Lys Ile Glu Pro Leu Gly Val Ala
Pro Thr Lys Ala Lys Arg Arg Val Val Gln Arg
Glu Lys Arg Ala Val Gly Ile Gly Ala Leu Phe
Leu Gly Phe Leu Gly Ala Ala Gly Ser Thr X
```

Kal-B:
```
    Z Leu Thr Val Gln Ala Arg Gln Leu Leu Ser
  Gly Ile Val Gln Gln Gln Asn Asn Leu Leu Arg
  Ala Ile Glu Ala Gln Gln His Leu Leu Gln Leu
  Thr Val Trp Gly Ile Lys Gln Leu Gln Ala Arg
  Ile Leu Ala Val Glu Arg Tyr Leu Lys Asp Gln
  Gln Leu Leu Gly Ile Trp Gly Cys Ser Gly Lys
  Leu Ile Cys Thr Thr Ala Val Pro Trp Asn Ala
  Ser Trp Ser Asn Lys Ser Leu Glu Gln Ile Trp
  Asn Asn X
```

X represents homoserine lactone or its hydrolysis or amidation products, which is a result of CNBr cleavage. Y is Met-Arg or Arg and Z is Met-Thr or Thr. Other subsets and analogs of the two sequences set forth above which have immunological activity are within the scope of the invention. Such subsets may be generated by replacing up to three amino acids with other natural or synthetic amino acids known to those skilled in the art. Analogs are generated by replacing up to three amino acids with other natural or synthetic amino acids known to those skilled in the art by recombinant or chemical methods, such that the resulting polypeptide retains at least substantially the same immunoreactivity. Analogs include subsets and sequences which have up to three amino acyl residues within the sequence deleted.

Additionally, we have discovered that expression of a Hind III-BamH I deletion mutant of Kal-10 produces a truncated ENV polypeptide with excellent immunoreactivity. Additionally, the truncated polypeptide is expressed in high yield, and may constitute about 5% of the total cellular protein in an induced culture. Use of this clone simplifies the purification of immunoreactive polypeptides which are ideal for stimulating the production of antibodies for use in diagnostic assays.

## EXAMPLES AND PREPARATIONS

The descriptions in the following examples and preparations will be clear to those skilled in the art of molecular biology or biochemistry. More detailed descriptions of generally used techniques for nucleic acid manipulation such as treatment with DNA polynucleotide kinase or DNA ligase, separation by electrophoresis, ethanol precipitation, electroelution, and other methods may be found in commonly used texts. See, for example, "Molecular Cloning, A Laboratory Manual" by T. Maniatis, et al., Cold Spring Harbor Laboratory (1982).

The cleavage of DNA by restriction enzyme digestion was carried out according to the current recommendations of the commercial suppliers.

## PREPARATION 1

Parental Plasmids

The plasmid pBR322 is about 4,400 nucleotide bases (4.4 Kb) in length and carries genes for resistance to the antibiotics ampicillin (Amp) and tetracycline (Tet). Its derivation and characterization have been described (F. Bolivar et al., Gene, 2, 95-113 (1977); Locus pBR322 GENBANK, DNA Database, Release 34 (1985)). The plasmids pUC8 and pUC9 each contain a copy of the Lac promoter and the amino terminus of the gene coding for the enzyme β-galactosidase. Into the amino terminal region of this gene is inserted a "cassette" comprising a DNA sequence recognized by several useful restriction enzymes. The plasmid pUC8 and pUC9 differ only in the orientation of this "cassette" and thereby facilitate the manipulation of DNA fragments during cloning. A description of the construction and characterization of the pUC plasmids has been published (J. Vieira and J. Messing, Gene, 19, 259-268 (1982)).

89821                                                    25160-FF

The E. coli Trp promoter-operator, the entire nucleotide sequence of which has been determined and analyzed (C. Yanofsky, et al., Nucleic Acids Res., 9, 6647-6668 (1981); Locus ECOTRP, GENBANK, DNA Database, Release 34 (1985)), was the source of the Trp promoter DNA fragment utilized. The particular plasmid employed was pNO342 which contains a 0.45 Kb fragment of E. coli Trp promoter-operator DNA from the closest Pvu II site upstream of the Trp promoter to the first Hinf I site, located in the TrpE gene. To construct pNO342, the Hinf I site on the Trp DNA was filled in and both ends (the Pvu II end and the filled-in Hinf I) then ligated to a Hind III ten base pair-long linker before insertion of the modified DNA fragment into the Hind III site of pBR322. The Hind III site nearest the EcoR I site preceding the Trp promoter of the pBR322 parent was then destroyed by cutting with Hind III, filling in the ends and religating. The Trp promoter-operator DNA could thus be removed from the resultant plasmid by digestion with EcoR I and Hind III. This final construct, pNO342, was obtained from Dr. Neil Osheroff, Biochemistry Department, Stanford University, Palo Alto, California 94305.

The DNA containing the transcription termination sequence utilized in the constructions described herein was derived from plasmid pVV202t, which was purchased from the laboratory of C. Yanofsky, Department of Biological Sciences, Stanford University, Palo Alto, California 94305. A 0.37 Kb BamH I fragment from pVV202t encompasses 325 base pairs from the transcription termination region of the E. coli rpoC gene (C. Squires, et al. Nucleic Acids Res., 9, 6817-6840 (1981); Locus ECORPLRP, bases #11226-11551 GENBANK Database, (October, 1984)). The TrpLE gene was obtained from plasmid pVV1, which has been previously described (B.P. Nichols, and C. Yanofsky, Methods in Enzymology, 101, 155-164 (1983)), and was purchased from the laboratory of C. Yanofsky.

Plasmid pBenn#20 DNA was used as the source of AIDS viral DNA for the various plasmid constructions. Derivation of pBenn#20 is as follows: a T lymphocyte culture (A3.01) (T. Folks et al., Proc. Nat. Acad. Sci. USA, 82, 4539-43 (1985)) was infected with the AIDS virus NA-2 (M. Martin, et al., manuscript in preparation). Nine days postinfection, the total cellular DNA was isolated from the infected culture. The high molecular weight cellular DNA (10 μg) was cleaved with restriction enzyme BamH I. 5 μg of the cleaved cellular DNA was ligated to 1 μg of BamH I-cleaved lambda J1 DNA (Nature, 308, pp 856-858, (1984)). The in vitro packaged ligated DNA was then used to infect E. coli cells. Recombinant phage containing AIDS proviral DNA were identified by means of in situ plaque hybridization, using a radioactively labelled AIDS viral cDNA as the hybridization probe. A positive phage plaque was identified. Analysis of DNA purified from the recombinant phage particles showed an insert of 17 Kb which included 8 Kb of the viral genome (the left long terminal repeat sequence, the GAG genes, the polymerase gene and three fourths of the envelope gene) as well as 9 Kb of the cellular flanking sequence. The 17 Kb insert was subsequently transferred to plasmid pBR322 via the unique BamH I site. The progeny plasmid was designated pBenn#20. Plasmid pBenn#20 is publicly available through Dr. Malcolm Martin, National Institutes of Health, Bethesda, Maryland.

PREPARATION 2

Oligonucleotide Synthesis

The protected deoxyribonucleosides were prepared as described by Ti, et al., J. Am. Chem. Soc., 104, 1316 (1982). All oligonucleotides may be synthesized by the solid phase phosphoramidite triester approach (M.H. Caruthers, et al., in "Gene Amplification and Analysis" T. Papas, M. Rosenberg, J. Chirikjian, Eds., Elsevier, New York, pp. 1-26 (1983)). Each chain can be built up using successive cycles of deprotection and condensation using 20 molar equivalents of 5'-O-N protected deoxynucleoside- 3'-O-methyl-N-morpholino phosphoramidites (T. Dorper and E.L. Winnaker, Nucleic Acids Res., 11, 2575 (1983)) activated by tetrazole, oxidation, and blocking of unreacted sites. After completion of synthesis, the methoxy and the N-protecting groups are removed (Caruthers (1983) op. cit.) and the crude dimethoxytrityl oligonucleotide is purified by reversed phase HPLC. After treatment with 80% acetic acid the fully deprotected product is further purified by electrophoresis on a preparative 20% polyacrylamide gel containing 7 M urea. Purified oligonucleotides are labelled using $[\gamma-^{32}P]ATP$ and polynucleotide kinase. The size and purity is then confirmed by electrophoresis on a 20% polyacrylamide/7 M urea gel.

## EXAMPLE 1

### Preparation of TrpLE plasmid

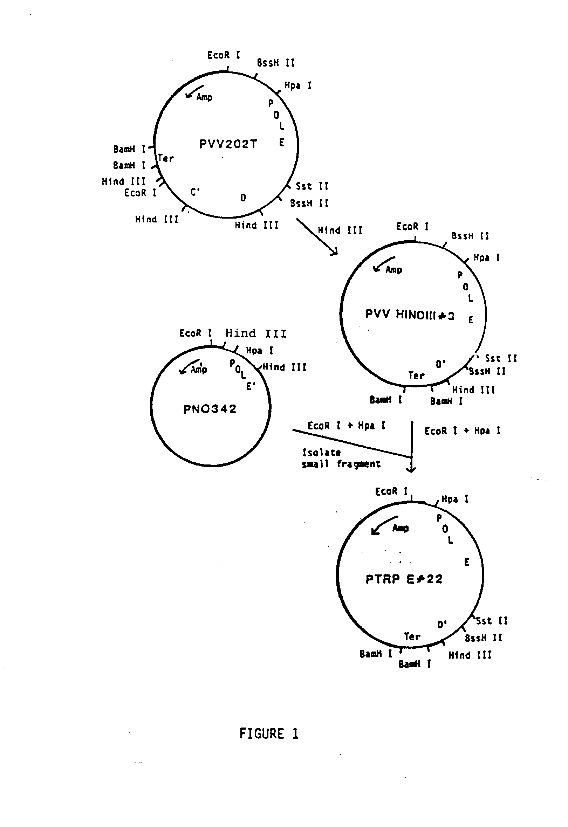

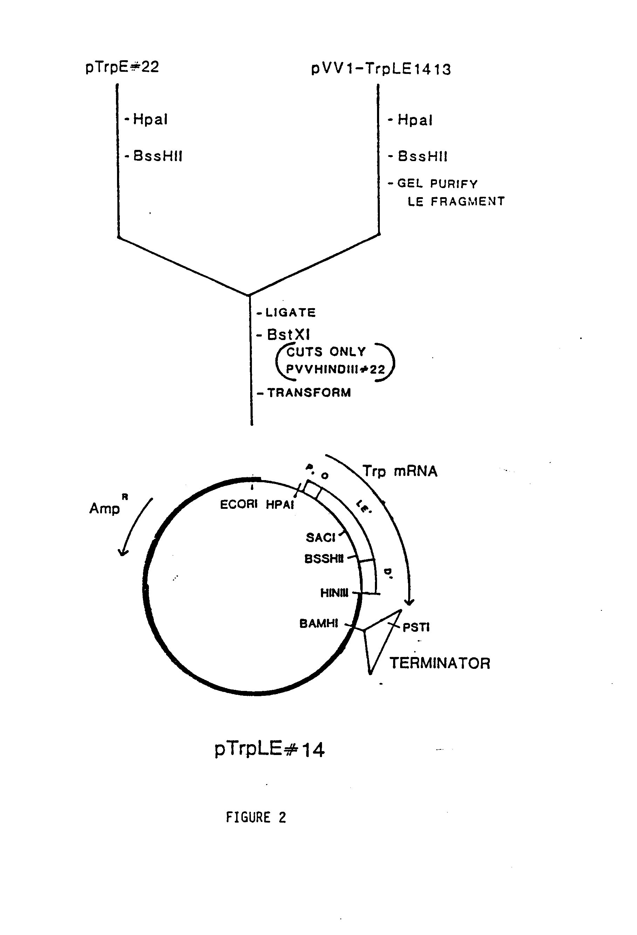

The construction of the plasmid cloning vector pTrpLE-#14 was a multi-step procedure (Figures 1 and 2). In the first step the plasmid pVV202t, which contains both the Trp E and D genes, as well as a portion of the TrpC gene, was treated to create a unique Hind III site. After digestion of 1.0 µg of pVV202t DNA with Hind III, phenol-CHCl$_3$ extraction and ethanol (EtOH) precipitation, 40 ng of the redissolved DNA was self-ligated with T4 DNA ligase and the products transformed into E. coli Hb101. Ampicillin resistant colonies were screened for the presence of a unique Hind III site between the start of the TrpD gene and the terminator; pVV-ΔHind III-#3 had the desired site.

It was desirable to have a plasmid with a unique BssH II restriction site in the TrpE gene for further cloning. Plasmid pVV-ΔHind III-#3 was found to have one or more BssH II restriction sites upstream of the Trp promoter in addition to the BssH II site in the TrpE gene. Consequently, the approximately 2.5 Kb fragment (EcoR I to Hpa I) of pVV-ΔHind III-#3 which contained part of the trp promoter, was replaced with a functionally analogous 282 bp EcoR I to Hpa I fragment from plasmid pNO342. The 0.28 Kb EcoR I to Hpa I fragment from pNO342 was isolated by electroelution from a 5% polyacrylamide gel after digestion of 5 µg of DNA. Then 10 ng of this DNA fragment were ligated with 80 ng of pVV-ΔHind III-#3, which had been previously cleaved with EcoR I and Hpa I. The products were transformed into E. coli Hb101 cells and the ampicillin resistant colonies were screened by digestion with BssH II to identify the desired clone, pTrpE-#22.

Replacement of the Trp promoter-Leader-E gene region of pTrpE-#22 with the analogous portion of pVV1 was the third and final step. DNA from pVV1 (100 µg) was digested with BssH II and Hpa I and electroeluted to produce fragments of about 6.0, 4.0, 1.8, 0.6, 0.57, and 0.54 Kb. The 0.57 Kb fragment containing the TrpLE gene was recovered by electroelution from the 4% polyacrylamide gel used to separate the digestion products. After phenol-CHCl$_3$ extraction and EtOH precipitation, 0.2 µg of the redissolved 0.57 Kb DNA fragment were ligated with 0.25 µg of the 5.1 Kb DNA fragment of pTrpE-#22, obtained by digestion with BssH II and Hpa I. The ligation products were digested with BstX I, because this recognition site resides in the portion of DNA deleted from the Trp promoter-operator in pVVl. Among the ampicillin resistant colonies, pTrpLE-#14 was identified as having the correct structure.

## EXAMPLE 2

Preparation of a clone coding for TrpLE·Sst II-HuGRF

A synthetic gene coding for $HuGRF_{Leu27}$ was designed to maximize the use of amino acid codons which are favored in highly expressed E. coli proteins, to minimize potential secondary structure in the resultant mRNA, to provide convenient restriction sites for screening the desired clone and to facilitate future alternatives in the DNA sequence. The synthetic gene sequence and the eighteen discrete oligodeoxynucleotides comprising the synthetic gene sequence are shown in the scheme below, and may be referred to in connection with the following more particularized discussion.

To facilitate cloning, the $HuGRF_{Leu27}$ gene was designed to be cloned in two segments; the A segment, comprised of ten oligodeoxynucleotides (Al through Al0), coding for the amino terminal portion of $HuGRF_{Leu27}$, and the B segment, comprised of eight oligodeoxynucleotides, coding for the carboxy terminal portion of the peptide, as illustrated in the following scheme:

```
                                 Met Tyr Ala Asp Ala Ile Phe Thr
     Sac II                      Nde I
       A1                         A3                      A5
    (C)CGC GGG ATG GTC CAT ATG TAC GCT GAT GCT ATA TTC ACT
          CC TAC CAG GTA TAC ATG CGA CTA CGA TAT AAG TGA
       ↑    A2                        A4                      A6
    (BssH II compatible)

    Asn Ser Tyr Arg Lys Val Leu Glu Gln Leu Ser Ala Arg Lys
    A5          A7                      A9                    Hind III
    AAC TCT TAC CGC AAG GTT CTG GGC CAA TTA TCA GCA CGC A
    TTG AGA ATG GCG TTC CAA GAC CCG GTT AAT AGT CGT GCG TTC GA
    A6                      A8                      A10
```

```
     Leu Leu Gln Asp lle Leu Ser Arg Gln Gln Gly Glu Ser Asn
B1        Pst I             B3    Sal I               B5
AG CTT CTG CAG GAC ATT CTT AGT CGA CAA CAG GGC GAA TCT AAC
    A GAC GTC CTG TAA GAA TCA GCT GTT GTC CCG CTT AGA TTG
    B2                              B4
```

```
Gln Glu Arg Gly Ala Arg Ala Arg Leu STOP
B5           B7                            Bcl I (BamH I
CAG GAG CGT GGC GCC AGA GCC AGA CTG T      compatible)
GTC CTC GCA CCG CGG TCT CGG TCT GAC ACTAG
B6                       B8
```

To assemble the A segment, each of the ten oligodeoxynucleotides (A1-A10) was individually phosphorylated. 50 pm of each of the ten phosphorylated oligodeoxynucleotides were then combined in a single tube. One tenth volume (20 μl) of 3 M sodium acetate (NaOAc) solution and 4 volumes (800 μl) of absolute EtOH were added with mixing. The solution was chilled for 15 minutes at -70°C and then centrifuged at 12,000 x G at 4°C for 15 minutes. The supernatant was removed and the pellet dried in vacuo. The dried pellet was then resuspended in 48 μl of 66 mM Tris-HCl (pH 7.5), 6.6 mM MgCl$_2$, 10 mM dithiothreitol, and 0.1 mM ATP. To anneal the oligonucleotides, the solution was heated to 40°C and then allowed to cool slowly over a 4 hour period to 20°C. 5 units of T4 DNA ligase were then added and the solution incubated for 12 hours at 20°C. The annealing and ligation reaction was repeated, and the ligated mixture phenol extracted and EtOH-precipitated. The dried, precipitated DNA was resuspended in 50 μl of sterile distilled water, and 35 μl of the ligated oligodeoxynucleotides were cleaved with 100 units each of Hind III and Sst II. The reaction mixture was incubated at 37°C for 4 hours and EtOH-precipitated. The Sst II/Hind III-HuGRF-A gene segment was gel purified from unreacted and partially ligated DNA fragments by preparative electophoresis on a 6.7% polyacrylamide gel.

The recovered DNA is referred to herein as the Sst II/Hind III-HuGRF-A gene segment.

Sixty-two ng of the plasmid pTrpE-#22 DNA which had previously been cleaved with BssH II, Hind III and Sst II was ligated along with about 6.7 ng (0.14 pm) of the Sst II-HuGRF-A gene segment in a final volume of 20 μl with 1.0 units T4 DNA ligase in 66 mM Tris-HCl (pH 7.5), 6.6 mM MgCl$_2$, 10 mM dithiothreitol, 0.1 mM ATP, for 16 hours at 12°C. The ligated DNA mixture was used for transformation of E. coli strain Hbl01. Transformants were selected for ampicillin resistance on L broth plates containing 50 μg/ml ampicillin. After screening 24 independently isolated colonies, eight were found to contain plasmid DNA with the additional Nde I site from the insert as well as the expected Sst II to Hind III insert of about 76 bp. Base sequence analysis according to Maxam and Gilbert verified that one of these had the expected sequence. This plasmid was designated pTrpE·Sst II-HuGRF$_{Leu27}$-A.

To assemble the remainder of the HuGRF$_{Leu27}$ gene, six of the eight oligodeoxynucleotides comprising the B segment (B2-B7) were individually phosphorylated. Phosphorylations were terminated by heating to 70°C for 10 minutes. Oligodeoxynucleotides B1 and B8 were not phosphorylated initially in order to prevent self-ligation at the termini. Two hundred picomoles of each of the phosphorylated oligodeoxynucleotides (B2-B7) were combined with 200 pm each of unphosphorylated B1 and B8. The oligomers were precipitated, annealed, and ligated as described above. The HuGRF-B gene segment was purified from unreacted and partially ligated DNA fragments by preparative electrophoresis on a 7.5% polyacrylamide gel. The recovered DNA was 5'-phosphorylated.

89821                                                           25160-FF

For the assembly of the A and B gene segments of HuGRF$_{Leu27}$, five micrograms of plasmid pTrpE·Sst II-HuGRF$_{Leu27}$-A was partially digested with 5 units of the restriction endonuclease BamH I. The reaction was terminated by phenol extraction and the DNA EtOH precipitated. The recovered DNA was digested to completion with ten units of the restriction endonuclease Hind III. The mixture was electrophoresed in a 0.7% agarose gel in order to resolve the partial cleavage products. The largest (6.216 Kb) fragment was excised and electroeluted. The recovered DNA was phenol extracted twice and EtOH precipitated.

Sixty ng of the 6.2 Kb Hind III and BamH I- cleaved pTrpE·Sst II-HuGRF$_{Leu27}$-A vector DNA was ligated along with an estimated five fold molar excess of the 72 bp HuGRF-B gene segment. The ligated DNA mixture was used for transformation of E. coli strain Hbl01. Transformants were selected for ampicillin resistance on L broth plates containing 50 µg/ml ampicillin. A number of the transformants were found to contain plasmid DNA with a Sst II to BamH I insert of about 0.5 Kb (the insert/terminator junction does not reconstitute a BamH I site). Base sequence analysis according to Maxam and Gilbert, Proc. Nat. Acad. Sci. USA, 74, 560 (1977), revealed that one of the plasmids, designated pTrpE·Sst II-HuGRF$_{Leu27}$ had the desired HuGRF$_{Leu27}$ coding sequence.

Transfer of HuGRF(Leu27) to pTrpLE-#14

The 0.5 Kb Sst II to BamH I insert comprised of the assembled A and B gene segments of HuGRF$_{Leu27}$ in addition to the transcription terminator, was purified from pTrpE·Sst II-HuGRF$_{Leu27}$ DNA by preparative electrophoresis on a 5% polyacrylamide gel.

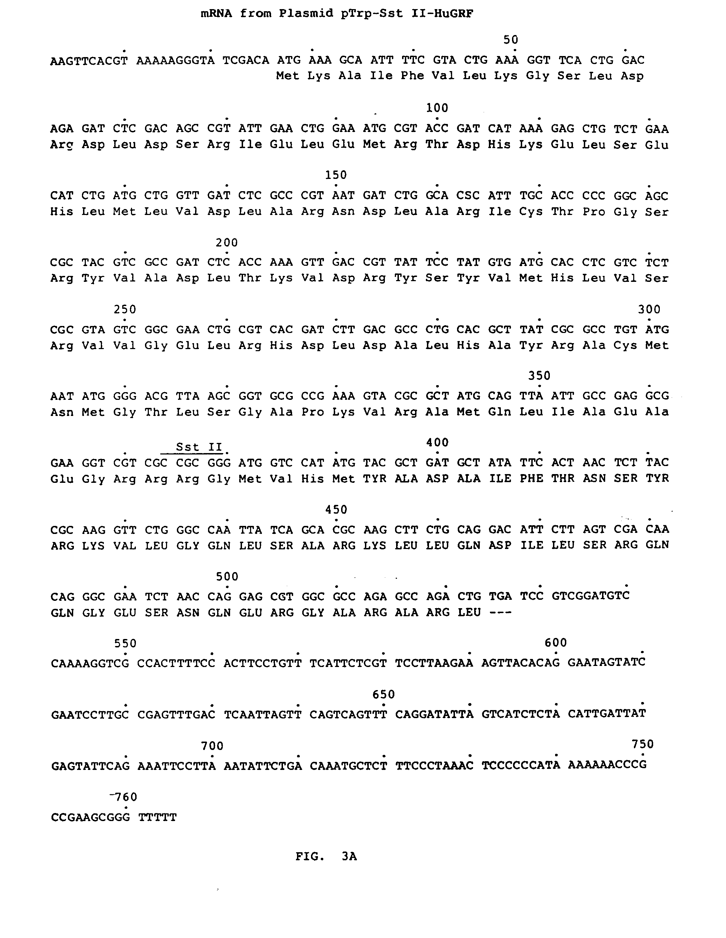

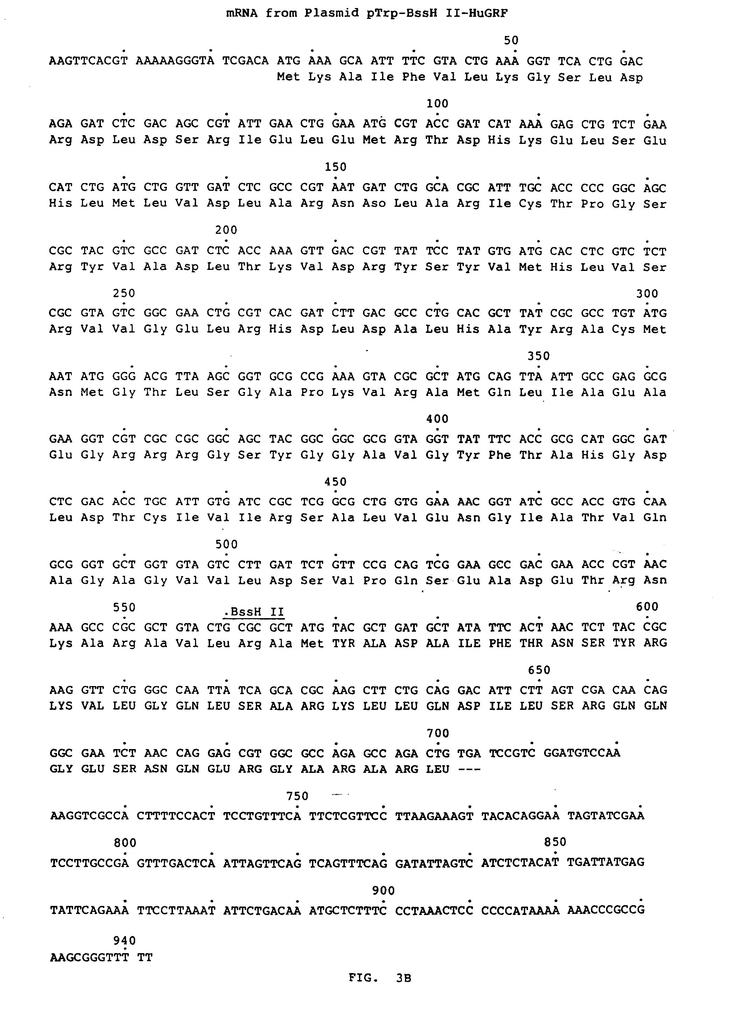

60 ng of BssH II, BamH I and Sst II- cleaved pTrpLE-#14 plasmid DNA was ligated with about a five fold molar excess of the 0.5 Kb Sst II to BamH I fragment. The ligated mixture was used for transformation of E. coli strain Hbl0l. Transformants were selected for ampicillin resistance on L broth plates containing 50 µg/ml ampicillin. After screening 12 independently isolated colonies, 2 were found to contain plasmid DNA with the correct Sst II to BamH I fragment. Base sequence analysis according to Maxam and Gilbert, Proc. Nat. Acad. Sci. U.S.A., 74, 560 (1977), verified that the plasmid pTrpLE·Sst II-HuGRF$_{Leu27}$ had the desired sequence (Figure 3A).

## EXAMPLE 3
Construction of pTrpLE·BssH II-HuGRF(Leu27)

Similarly, one may construct a gene to express TrpLE-HuGRF$_{Leu27}$ using the unique BssH II site rather than the Sst II site in the TrpLE gene.

Eight of the ten oligodeoxynucleotides comprising the A segment of HuGRF$_{Leu27}$ (A2 through A9) were individually phosphorylated. The reaction was terminated by heating to 70°C for 10 min. Oligodeoxynucleotides A1 and A10 were not initially phosphorylated to prevent self-ligation at the termini. 200 picomoles (pM) of each of the phosphorylated oligodeoxynucleotides (A2-A9) were combined with 200 pM of each of A1 and A10. The oligomers were EtOH precipitated, annealed and ligated. The HuGRF-A gene segment was purified from unreacted and partially ligated DNA fragments by preparative electrophoresis on a 7.5% polyacrylamide gel. The 5' ends of the recovered HuGRF-A segment are then phosphorylated and ligated to plasmid pTrpE-#22, previously cleaved with BssH II and Hind III. The ligation mixture was used to transform E. coli Hbl0l. Transformants were screened for the presence of an

89821                                                      25160-FF

additional Nde I restriction site. The desired clone was designated pTrpE•BssH II-HuGRF-A.

To complete the HuGRF$_{Leu27}$ gene sequence, the HuGRF-B gene segment as well as the transcriptional terminator were utilized from pTrpE•Sst II-HuGRF$_{Leu27}$. Plasmid pTrpE•Sst II-HuGRF$_{Leu27}$ was cleaved at the unique BamH I and Hind III sites. The 0.4 Kb Hind III to BamH I fragment was purified by gel electrophoresis. Plasmid pTrpE•BssH II-HuGRF-A was cleaved with BamH I and Hind III and the largest fragment purified from a 0.7% agarose gel after electrophoresis. These two purified Hind III to BamH I fragments were ligated and transformed into E. coli Hb101. Plasmid DNA from the transformants was screened for the presence of 3 Pst I sites (one Pst I site is conferred by the insert). By this technique plasmid pTrpE•BssH II-HuGRF$_{Leu27}$ was identified. Base sequence analysis (Maxam and Gilbert) revealed an unexpected single base pair deletion at the start of the BssH II-HuGRF-A gene segment. Thus, the sequence GCG CGA TG--- was found rather than the expected sequence GCG CGG ATG--. This resulted in the alteration of the triplet reading frame for the remainder of the HuGRF$_{Leu27}$ gene, which was repaired in the next step.

The replacement of the TrpE DNA segment of plasmid pTrpE•BssH II-HuGRF$_{Leu27}$ by the TrpLE DNA segment was achieved by joining portions of two previously described plasmids via a synthetic oligonucleotide. This oligonucleotide was designed to replace the HuGRF-A DNA sequence between the BssH II and Nde I sites in order to restore the proper translational reading frame for HuGRF$_{Leu27}$ synthesis. To provide the heterologous HuGRF$_{Leu27}$ coding gene, 12.5 µg of pTrpE•BssH II-HuGRF$_{Leu27}$ DNA was cleaved with Nde I and BamH I and the desired 0.5 Kb fragment isolated from an agarose gel. Next, the self-complimentary synthetic oligonucleotide TAGCGCGC was treated with T4 kinase, and 300 ng was ligated to 0.5 ng of the isolated fragment. This reaction mixture was next heated to 60°C for 15 minutes to inactivate the kinase, and then cleaved with BssH II to convert the Nde I end of the 0.5 Kb Nde I-BamH I fragment into a BssH II site, as illustrated below:

```
                                 Nde I              BamH I
                                   ↓                  ↓
             GCG CG(G) ATG GTC CAT ATG TAC----GGATCC---
             CGC GC(C) TAC CAG GTA TAC ATG----CCTAGG---
                                     I←    0.5 Kb    →I
                                   ↓
                                 + BamH I
                                 + Nde I
                                   ↓
                        TATGTAC-----------G
                        ACATG-----------CCTAG
                                   I
  TAGCGCGC                         I
    CGCGCGAT                       I
       + T4 Kinase                 I
         ↓                         I
       ↓←----------------------
         ↓
  TAGCGCGCTATGTAC---------------G
    CGCGCGATACATG---------------CCTAG
       ↓
    + BssH II
       ↓
  CGCGCTATGTAC-------------G
     GATACATG-------------CCTAG
```

The TrpLE coding gene was provided by digestion of plasmid pTrpLE-#14 with BssH II and BamH I. A ligation between 100 ng of the 0.5 Kb BssH II-BamH I fragment and 100 ng of the double digested pTrpLE-#14 DNA was performed. The ligation products were transformed into competent E. coli Hbl01 cells and plated onto ampicillin plates. Restriction enzyme screening of DNA from the resulting colonies by digestion with BssH II plus BamH I or with Pst I identified 7 out of 24 candidates as having

-45-

the anticipated structure. The DNA sequence of plasmid pTrpLE·BssH II-HuGRF$_{Leu27}$-#5 was confirmed by DNA sequence analysis (Figure 3B).

### EXAMPLE 4
### Expression and Purification of LE-HuGRF(Leu27)

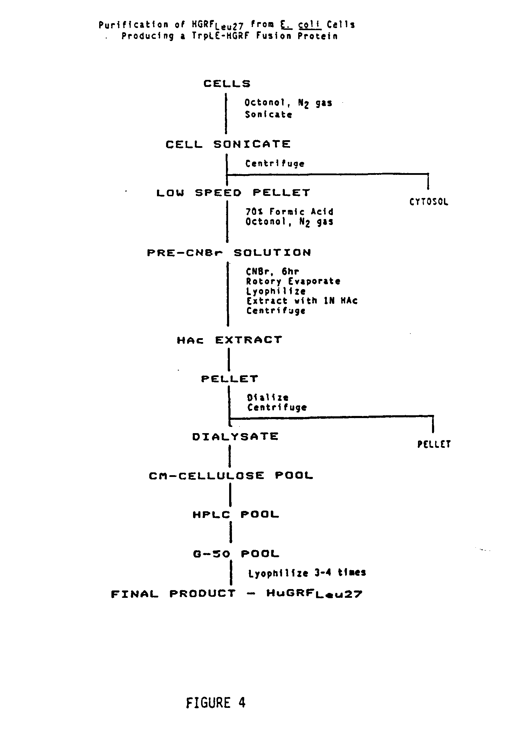

The procedure described herein can be used to purify HuGRF from the LE-HPGRF$_{Leu27}$ clones which utilize either the BssH II or Sac II fusion site to the TrpLE protein. Before beginning, the extent of induction can be monitored by analysis of an aliquot of the washed cell pellet on a stained SDS gel. There is no difference in the level of expression or the purification steps up to the point of dialysis with either clone (Figure 4). During dialysis, the BssH II clone deposits a large precipitate, whereas the Sac II clone forms a smaller precipitate. Centrifugation of the BssH II dialysate thus leaves a larger pellet which must be thoroughly washed to fully recover the LE-HPGRF$_{Leu27}$.

### Induction of TrpLE-HuGRF

In this case, a starter culture of 50 ml of LBr culture media (reference to the preparation of the LBr and M9 media described here can be found in J. H. Miller (1972) "Experiments in Molecular Genetics," p. 431) containing 50 μg/ml of ampicillin and 100 μg/ml of tryptophan was inoculated with the E. coli cells containing the plasmid, i.e., pTrpLE·BssH II-HuGRF$_{Leu27}$, and grown overnight at 37°C with vigorous shaking to a final A$_{550}$ of about 6. Four liters of M9 culture media for production were pre-warmed to 37°C and seeded with 40 ml of the LBr starter culture to give a final A$_{550}$ of about 0.6. The culture is then grown with vigorous shaking to an A$_{550}$ of about 0.2 to 0.5, whereupon 4 ml of a 10 mg/ml solution of indole acrylic acid (IAA) in EtOH is added to give a final concentration

of 10 μg/ml solution of IAA. Growth is then continued with good aeration for about 16 hr to a final $A_{550}$ of about 5 (typically, about 5 to 10). The addition of IAA during growth in M9 media is optional since it increases only the kinetics of product accumulation and not the final yield. An alternate and equally effective method of cell growth is to substitute LBr for M9, in which case the addition of IAA is essential for adequate product yields. The cells were concentrated by centrifugation, resuspended in 500 ml (typically 1/10 to 1/20 of the original culture volume) of 50 mM Tris-HCl, pH 7.1, and re-pelleted. The washed cells may be conveniently stored at -20°C at this stage.

Sonication

The washed cells are lysed by sonic disruption. Cells were resuspended in 900 ml (typically 20-100 $A_{550}$ units per ml) of 50 mM Tris-HCl, pH 7.5, 0.1 mM EDTA, 1 mM phenylmethylsulfonyl fluoride (PMSF) (this buffer solution will be abbreviated "TE-PMSF"), cooled to 4°C, a few drops of octanol added and $N_2$ gas bubbled through the solution. These steps serve to minimize heating of the sample and oxidation of methionine residues during sonication. The suspension was sonicated using a model W220F Heat Systems-Ultrasonics, Inc. sonicator (equipped with a 3/4" horn) according to the manufacturer's recommendations. The sonicator was typically operated only 50% of the total process time to avoid overheating.

Centrifugation

The cell lysate was centrifuged for 15 min at about $3600 \times g_{max}$ to pellet the pTrpLE-HuGRF$_{Leu27}$ and the supernatant discarded. The pellet was resuspended in 450 ml TE-PMSF (typically 40-80 $A_{550}$/ml) and the pellet collected by centrifugation as before.

## EXAMPLE 5

### Purification of HuGRF(Leu27)

Cleavage of the methionine residues in the TrpLE-HuGRF$_{Leu27}$ fusion protein with CNBr causes release of the desired heterologous HuGRF$_{Leu27}$ protein, which can be purified as described below.

### CNBr Treatment of Pellet

The washed pellet of TrpLE-HuGRF$_{Leu27}$ was resuspended by gently stirring or swirling it in 60 ml 70% formic acid (about 20 mg/ml total protein; typically material from 1000 A$_{550}$ units of cells is dissolved in 3 ml). A few drops of octanol were added and N$_2$ bubbled through the solution for 20 minutes before adding 5.5 g CNBr. This reaction was allowed to proceed for 6 hours at 25°C before the addition of an equal volume of 50:50 MeOH:H$_2$O in order to facilitate the subsequent concentration step. The volatile excess CNBr, water and organic solvents were removed by rotary evaporation until the volume was reduced by one-half. Additional 50:50 MeOH:H$_2$O was mixed with the sample and subsequently removed by rotary evaporation. After 2-4 repetitions of this process, the bulk of the formic acid and CNBr had been removed. The sample was then evaporated to dryness, redissolved in 200 ml of water and lyophilized.

### Acetic Acid Extraction

The lyophilized sample was dissolved in 80 ml 1N acetic acid, and extracted for at least 30 minutes at 25°C. After centrifugation for 10 minutes at 12,000 x g$_{max}$ the supernatant was recovered and the pellet reextracted with 70 ml 1 N acetic acid as before. The pellet fraction was discarded and the supernatants, which contain the HuGRF$_{Leu27}$, were combined.

### Dialysis

The 1N HOAc extract was next placed into dialysis tubing having a molecular weight cutoff of 3500 and dialyzed for a total of 18 h against four changes of 10 mM NaOAc, pH 5.0, 4 L each. The first half of the dialysis was performed at 25°C, and the second half at 4°C. The precipitate which formed was removed by centrifugation for 10 min. at 10,000 x $g_{max}$. If warranted, the pellet was washed with buffer to maximize recovery of HuGRF in the soluble fraction.

CM-Cellulose Chromatography

The clarified dialysate was then passed through a 50 ml bed volume CM-cellulose column (2.5x10 cm) at 1.5 ml/min in a cold room (0-4°C). After washing with 50 mM NaOAc, pH 5, a gradient from 50 to 500 mM NaOAc, pH 5 was used to elute the HuGRF$_{Leu27}$. The peak of HuGRF$_{Leu27}$ could be determined by immunoblotting (western analysis) of the column fractions.

HPLC

Pooled fractions from the CM-cellulose column were filtered through a milex-GV 0.22 μm filter (Milipore) and applied at 4 ml/min directly onto an 0.8x25 cm HPLC column packed with Vydec C18 TP201 N-capped (10 μm) 300 Å pore beads which had been equilibrated in solvent A (0.1% trifluoroacetic acid in water). The column was then washed with 75:25 solvent A:solvent B (0.1% trifluoroacetic acid in acetonitrile) until the baseline returned to zero. The HuGRF$_{Leu27}$ was then eluted with a gradient running from 25 to 35% solvent B over a period of 30 min. The major peak in the chromatogram, which contained the HuGRF, eluted after about 26 minutes and was collected. The solvent was removed by 3-4 cycles of rotary evaporation from water.

Gel Filtration

As a final purification step, and in order to replace any remaining traces of $CF_3CO_2-$ with Cl-, the dried sample was dissolved in 4 ml of 25 mM HCl and applied to a 25 ml column (2.5x5cm) of Sephadex G-50 which had been equilibrated in the solvent. The column effluent was monitored by absorbance at 280 nm and the void volume peak containing the HuGRF was pooled. This material was lyophilized several times from water until the pH of the solution exceeded 5. A white, fluffy material was obtained.

## EXAMPLE 6

### Characterization And Biological Activity of HuGRF(Leu27)

A purity of greater than 95% for the final HuGRF$_{Leu27}$ product was demonstrated by SDS gels (Burr and Burr, Meth. Enz., 96, 239-244 (1983)) either stained for total protein or visualized by western blotting with anti-GRF antisera (Figure 9). In addition, an HPLC profile was obtained (Figure 10). The measured dry weight of the final product and the theoretical weight calculated from the total amino acid content agreed to within 5%. Further, the amino acid composition and sequence agreed with the predicted results.

The 44-amino acid peptide GRF induces secretion of growth hormone from the anterior pituitary gland. The mechanism of action involves a receptor-mediated activation of adenylate cyclase and elevation of intracellular cyclic AMP (Labrie, F., B. Gagne, and G. Lefevere, Life Sciences, 33, 2229-2223 (1983)). The biological potency of the HuGRF$_{Leu27}$ was therefore demonstrated by its ability to activate adenylate cyclase, by its specific binding to pituitary cells, and by its ability to stimulate the release of GRF from pituitary cells.

### Adenylate Cyclase Enzyme Activation Assay

To assay adenylate cyclase activation by GRF we chose bovine pituitary glands. Pituitary membranes were lyophilized and enzyme activity was assayed in accordance with established procedures (Alvarez, R., and J.J. Bruno, Proc. Nat. Acad. Sci. USA, 74, 92-95 (1977)). The HuGRF$_{Leu27}$ had an EC$_{50}$ of 2 μM. The shape of the response curve and the molar potency were similar to that of authentic HuGRF-NH$_2$. We concluded that the peptide produced by recombinant DNA techniques has definite biochemical activity.

Binding Assay

The specific binding of GRF to pituitary cells was determined as follows:

GH3 rat pituitary tumor cells, a somatotrophic cell line which was obtained from Dr. D. Gospodarowicz (University of California, San Francisco), were grown in Dulbecco's minimal essential medium (DMEM). Synthetic HuGRF-NH$_2$ was purchased from Peninsula Labs (San Carlos, CA) and radioactively labeled with Na[$^{125}$I] using Enzymobeads (BioRad) to a specific activity of 20,000 cpm/ng protein. Cells were grown to confluence in 24-well Costar trays washed with DMEM buffered at pH 7.4 with 20 mM HEPES and containing 0.1% bovine serum albumin (DMEM-BSA). Cells were incubated at 4°C for one hour with 2 x 10$^{-9}$ M [$^{125}$I]-HuGRF(1-44) in either the absence or presence of increasing concentrations of recombinant GRF analogs in a final volume of 250 μl DMEM-BSA. Cells were washed three times with DMEM-BSA, lysed with 0.1 N NaOH and the cell-associated radioactivity was determined in a gamma counter. Nonspecific binding was assessed in the presence of 10$^{-7}$ M synthetic HuGRF-NH$_2$ and did not exceed 15% of the specific binding.

In independent experiments it was established that GH3 cells possess specific receptors for HuGRF-NH$_2$. Binding was saturable and reversible. Scatchard analysis of this data revealed about 20,000 binding sites per cell with an apparent K$_d$ for hpGRF-(1-44) of about 1.0 x 10$^9$ M$^{-1}$.

The recombinant human and porcine GRF were almost equipotent with synthetic human GRF (2 to 3-fold reduced affinity) for their receptor binding ability.

Growth Hormone Secretion Assay

The ability of GRF to stimulate the release of growth hormone from pituitary cells was also assayed. Normal rat anterior pituitary cells were established in primary cultures as follows: Male adult Sprague-Dawley rats (250 g) were sacrificed by decapitation. The anterior pituitary was dissected, placed in DMEM containing 1% BSA and minced into 1 mm pieces. After several washes with DMEM-BSA, the tissue was incubated at 37°C for 35 minutes with 0.2 U trypsin/ml and 1 mg/ml DNAase/ml with gentle shaking. Tissue fragments were then further dispersed by physical shearing with a Pasteur pipette. Large fragments were allowed to settle and were removed. Cells in the supernatant were then pelleted, washed and plated for tissue culture for 4 days in DMEM + 10% FCS before an experiment. The average yield of viable cells was approximately $2 \times 10^6$ cells per pituitary. Cells were plated in 24-well dishes and incubated with GRF or analogs for 3 hours at 37°C.

The concentration of GH in the medium was determined by a solid phase RIA using reagents from the NIH Pituitary Agency. Ninety-six well polyvinylchloride plates were coated overnight at 4°C with rabbit anti-monkey Ig antibodies (Pel-Freez) at 0.1 mg/ml in phosphate buffered saline (PBS). Plates were countercoated with a 3% BSA solution in PBS for at least one hour at 37°C. Plates were washed and incubated with monkey anti-rat GH antiserum (1/10,000 final dilution) for 2 hours or more at 37°C. After several washes with PBS-0.1% BSA, plates were incubated at 37°C for 2 hours with 20 ng/ml $[^{125}I]$-GH in the presence or absence of unlabeled GH or dilutions of cellular conditioned medium.  After several washes, plates were dried and individual wells were cut out and counted in a gamma counter.

The recombinant human and porcine GRF appeared equipotent to and as efficacious as synthetic human GRF for the induction of GH release.


### EXAMPLE 7
#### Construction And Use Of pTrpLE-PGRF(Leu27)

Using an approach and methods similar to that described in Examples 2-3, plasmids were constructed in which either the unique BssH II site or the unique Sst II site of the pTrpLE-#14 vector was utilized to encode for a fusion protein between the TrpLE protein and the $PGRF_{Leu27}$ analogue of PGRF.  This was accomplished by replacing that portion of the synthetic DNA encoding HuGRF located between the Sal I site within the HuGRF DNA segment and the BamH I site at the proximal end of the terminator DNA segment by synthetic DNA encoding PGRF. The replacement of the HuGRF synthetic DNA by the PGRF synthetic DNA was facilitated by first constructing plasmid pPS#8 in which the Sal I site within the HuGRF gene and the BamH I site just beyond its end were both unique.

#### Construction of vector plasmid pPS#8

The BamH I site in plasmid pTrpE-#22 which lies the farthest from the TrpLE gene was first removed by digesting the plasmid with BamH I and Sal I, EtOH precipitating the DNA, and treating the mixture with T4 ligase.  (BamH I cuts at each end of the terminator DNA segment, and Sal I cuts within the pBR322 DNA region and within the terminator, nearest the Hind III site.)  The ligation products were treated with Sph I (which cuts within the pBR322 DNA region) and transformed into competent Hbl01 cells.  One of the resulting plasmids, pVVΔHinΔSal#15, was shown to contain the 0.33 Kb BamH I to Sal I fragment from the transcriptional terminator region of pTrpE-#22 flanked by unique BamH I and Sal I sites. This fragment was in the opposite orientation in pVVΔHinΔSal#15 compared to pTrpE-#22, but it functions effectively in both orientations.

The Sal I site in pVVΔHinΔSal#15 was next removed by deleting the DNA between the Sal I and the Pvu II sites of the pBR322 portion of the vector. This was accomplished by digesting pVVΔHinΔSal#15 with Sal I and Pvu II and filling in the Sal I end by incubating with DNA polymerase I Klenow fragment. The resulting DNA was then cleaved with Sal I and transformed into Hb101 cells. The resulting plasmid, pPS#3, contained the expected deletion and lacked a Sal I site. Since the procedure described would be expected to regenerate a Sal I site, the lack of a Sal I site in pPS#3 probably reflects the increased transformation efficiency of circular DNA lacking a Sal I site, as compared with Sal I-cleaved linear DNA.

Next, the 1.35 Kb EcoR I to BamH I fragment encoding TrpLE-HuGRF$_{Leu27}$ was excised from pTrpLE·BssH II-HuGRF#5 and ligated with EcoR I and BamH I cleaved pPS#3 DNA. The ligation mixture was treated with BstX I, which cleaves only the pPS#3 DNA fragment not desired in the final construct, and transformed into competent Hb101 cells. The resulting plasmid, pPS#8, containing a gene encoding the TrpLE-HuGRF protein, with its Sal I site, followed by a transcriptional terminator DNA segment, also containing a Sal I site, followed by a unique BamH I site, followed by a transcriptional terminator DNA segment lacking a Sal I site.

Insertion Of Synthetic DNA Encoding PGRF

The three amino acid substitutions which distinguish PGRF from HuGRF all lie beyond the Sal I site in the

HuGRF$_{Leu27}$ gene sequence of pPS#8. Since the sequence beyond the BamH I site of pPS#8 contained a functional transcriptional terminator, synthetic DNA encoding PGRF was designed to replace the DNA between these two sites in pPS#8. Six oligonucleotides were synthesized, ligated, cleaved with Sal I and BamH I, and the 54 bp oligonucleotide illustrated below isolated from a 5% polyacrylamide gel:

```
     Arg Gln Gln Gly Glu Arg Asn Gln Glu Gln Gly Ala Arg
  C1                        C3                      C5
   T CGA CAA CAG GGC GCC CGT AAC CAG GAG CAG GGC GCC AGG
  (Sal I) GTT GTC CCG CGG GCA TTG GTC CTC GTC CCG CGG TCC
        C2                            C4
```

```
Val Arg Leu STOP
C5        Xba I
GTG CGG CTC TAG AG   (BamH I)
CAC GCC GAG ATC TCC TAG
   C6
```

This 54 bp fragment was ligated to pPS#8 which had been previously cleaved with Sal I and BamH I and the resultant clones screened for the presence of the unique Xba I site which was designed into the synthetic PGRF DNA. Plasmid pTrpLE·BssH II-PGRF#10 was thus identified, followed by experimental verification of the DNA sequence.

By using a similar approach with a vector analogous to pPS#8, but containing a HuGRF gene fused to the Sac II site of TrpLE, the plasmid pTrpLE·Sac II-PGRF was constructed.

Purification and Biological Activity of PGRF(Leu27)

Using the procedures described in Examples 4-6, E. coli cells (strain W3110 prototroph, ATCC No. 27325) containing plasmids encoding the TrpLE-PGRF$_{Leu27}$ fusion protein were grown, expression of the fusion protein induced, and the PGRF$_{Leu27}$ released by CNBr treatment

-55-

and purified. The purity and biological activity of the final product were comparable to that described for HuGRF$_{Leu27}$.

EXAMPLE 8

Construction and Use of a pTrpLE-BGRF(Leu27) Vector

Following the approach outlined above, the carboxy terminal encoding region of the HuGRF portion of plasmid pPS#8 was altered to encode the BGRF$_{Leu27}$ analog of BGRF. Since the five amino acid differences which distinguish BGRF from HuGRF$_{Leu27}$ all lie beyond the Hind III site in the HuGRF$_{Leu27}$ coding region of plasmid pPS#8, eight oligonucleotides were designed to encode the BGRF amino acid sequence and replace the DNA fragment between Hind III and BamH I. After these oligonucleotides were synthesized, phosphorylated, ligated and cloned into pUC8, cleavage with Hind III and BamH I produced the following 75 bp DNA fragment:

```
          Leu Leu Gln Asp Ile Leu Asn Arg Gln Gln Gly Glu Arg
      D1                      D3                            D5
   5' AG CTT CTG CAG GAC ATT CTT AAC CGA CAA CAG GGC GAA CGT
   3'    A GAC GTC CTG TAA GAA TTG GCT GTT GTC CCG CTT GCA
Hind III D2                                  D4
```

```
Asn Gln Glu Gln Gly Ala Lys Val Arg Leu STOP
D5                  D7                          BamH I
AAC CAG GAG CAG GGC GCC AAG GTG CGG CTC TAG AG
TTG GTC CTC GTC CCG CGG TTC CAC GCC GAG ATC TCC TAG
      D6                      D8
```

This fragment was next ligated into Hind III and BamH I cleaved plasmid pPS#8 to produce the desired plasmid, pTrpLE·BssH II-BGRF$_{Leu27}$#15.

The procedures described in Examples 4-6 were used to grow E. coli cells (strain W3110 prototroph, ATCC No. 27325) containing plasmid pTrpLE·BssH II-BGRF$_{Leu27}$#15, express the fusion protein, purify the

BGRF$_{Leu27}$ released by CNBr cleavage and test the biological activity. Results demonstrated activity comparable to HuGRF$_{Leu27}$ and PGRF$_{Leu27}$.

## EXAMPLE 9

Formulations

HuGRF$_{Leu27}$, BGRF$_{Leu27}$, or PGRF$_{Leu27}$ is dissolved or suspended in physiological saline at a concentration of about 1 to 5 µg/ml. Other pharmaceutically acceptable excipients may be added if desired. Two ml of the resulting solution is administered by intramuscular or subcutaneous injection one to four times per day. A typical mammalian dose is about 0.1 to 0.5 µg per Kg body weight per day.

Alternatively, HuGRF$_{Leu27}$, BGRF$_{Leu27}$, or PGRF$_{Leu27}$ may be dissolved or suspended in physiological saline and the solution or suspension installed in a controlled release device for continuous administration. A typical continuous release rate is from about 1 to about 5 µg per hour per Kg body weight.

## EXAMPLE 10

Construction of 3 open-reading-frame vectors

In order to construct a set of three vectors suitable for expression of any open reading frame as a fusion to the TrpLE protein, oligonucleotides encoding the BamH I recognition sequence GGATCC were inserted near the unique BssH II site of the TrpLE gene in each of the three possible translational reading frames relative to the TrpLE coding gene.

To provide a substrate for the ligation of these oligonucleotides, plasmid pPS#8 DNA (2 µg) (See Example 7) was cleaved at 50°C for one hour with 4 units of the restriction endonuclease BssH II. The reaction was terminated by extracting with phenol and the DNA EtOH precipitated. To fill the BssH II-generated 5' overhangs, the DNA was resuspended in 20 µl of buffer containing 40 mM $KHPO_4$ (pH 7.5), 6.6 mM $MgCl_2$, 1.0 mM 2-mercaptoethanol, 250 µM of each of the four deoxynucleotides. 1 unit of Klenow fragment (E. coli DNA polymerase large fragment) was added and the mixture incubated at room temperature for 15 minutes.

To construct the three reading frame vectors, the flush-ended BssH II-cleaved pPS#8 DNA was divided into three reactions. Each of the reactions contained 0.5 µg of DNA in ligation buffer [50 mM Tris-HCl (pH 7.8), 10 mM $MgCl_2$, 20 mM dithiothreitol], 100 µM ATP, 2 units of T4 ligase and a 100-fold excess of one of the three synthetic BamH I linkers (CGGATCCG, CCGGATCCGG or CCCGGATCCGGG). After overnight incubation at 12°C, the reaction mixtures were adjusted to 150 mM NaCl, 6 mM Tris-HCl (pH 7.9), and 6mM $MgCl_2$ and treated with 1 unit of BamH I. After 1 hour's incubation at 37°C the reactions were terminated by phenol extraction. DNA was recovered from each reaction by EtOH precipitation. Each precipitated DNA was resuspended in 10 µl of ligation buffer and treated with 1 unit of T4 ligase. The ligation mixtures were then used to transform competent E. coli Hb101 cells. Progeny clones, in which a BamH I site lies between the BssH II site of TrpLE (GCGCGC) and the terminator DNA segment, were designated as pSB8, pSB10 and pSB12. Nucleotide sequencing verified that these three vectors contain the appropriate BamH I linkers, according to the following scheme:

```
                      BamH 1
pSB8    ----CTG CGC GCG GAT CCG TCG ------
        ----Leu Arg Ala Asp Pro Ser ------
```

```
                                BamH I
pSB10   ----CTG CGC GCC GGA TCC GTC ------
        ----Leu Arg Ala Gly Ser Val ------

                                BamH I
pSB12   ----CTG CGC GCC CGG ATC CGT ------
        ----Leu Arg Ala Arg Ile Arg ------
```

EXAMPLE 11

Construction of Genomic Library for AIDS

The construction of a library of clones capable of expressing open reading frames of the AIDS virus fused to the TrpLE protein was achieved by ligation of a partial Sau3A digest of AIDS DNA into the BamH I site of the three open reading frame vectors, pSB8, pSB10, and pSB12. A partial Sau3A digest of AIDS DNA was used to increase the chances that large open reading frames, which may be interrupted by one or more Sau3A sites, will be represented intact.

Plasmid pBenn#20 (10 μg) was doubly digested with restriction enzymes BamH I and Sst I (10 units each) using the buffer conditions recommended for BamH I at 37°C for 90 minutes. The resulting DNA fragments were resolved on an 0.7% preparative agarose gel. An 8 Kb fragment which encompassed four fifths of the AIDS viral genome was eluted from the agarose gel and concentrated by EtOH precipitation. The resuspended DNA (in buffer containing 50 mM NaCl, 6 mM Tris-HCl (pH 7.5), and 5 mM MgCl$_2$) was divided into three reactions, each of which was digested with 0.1 or 0.01 units of Sau3A at room temperature for 15 min. After phenol extraction to terminate the reactions, the extent of Sau3A digestion was monitored by analyzing the cleavage products on an agarose gel. The DNA sample treated with 0.1 unit of Sau3A showed the appropriate extent of partial digestion and was chosen for subsequent ligation to the three open-reading frame vectors. Approximately 200 ng of the

-59-

partially Sau3A-digested DNA was mixed with 100 ng each of the three vectors that had been previously treated with BamH I and alkaline phosphatase in 30 µl of ligation buffer. One unit of T4 ligase was added to each of the reactions and the mixtures were incubated for 6 hours at 12°C. The ligated DNA from each of the three ligation reactions was then separately transformed into competent E. coli Hbl01 cells.

A total of approximately 400 clones were obtained from the initial transformation. Greater than 80% of these clones contained AIDS viral DNA inserts as evidenced by their positive hybridization to fragments of pBenn#20. Progeny clones were further screened for their ability to produce novel protein molecules that were immunoreactive with anti-AIDS virus sera obtained from AIDS patients (Figure 12). Nucleotide sequencing was performed on several clones to determine the sequences at each vector-insert junction. The results (Table 1, Figure 11) verified that the positive clones contained AIDS viral fragments inserted in the proper orientation and correct reading frames with regard to the transcription and translation of the LE carrier protein. Moreover, the origins and boundaries of the inserts within the AIDS viral genome were defined, using published AIDS viral DNA sequences as a reference. (S. Wain-Hobson, et al., Cell, 40, 9-17 (1985); R. Sanchez-Pescador, et al., Science, 227, 484-492 (1985); L. Ratner, et al., Nature, 313, 277-284 (1985); M.Muesing, et al., Nature, 313, 450-458 (1985)).

EXAMPLE 12

Construction of other specific AIDS viral clones

In addition to the AIDS genomic library described above, which was based on the insertion of random Sau3A fragments into the three open-reading-frames vectors, two

-60-

additional clones containing specific DNA fragments were constructed (Table 1, Figure 11).

The first specific construct utilized a pTrpLE-derived vector (designated pTrpLE-KAL). pTrpLE-KAL was constructed by cleaving pTrpLE-#14 with BssH II and BamH I, followed by the introduction of oligonucleotides to read as follows:

```
(former)
  BssH II                         Kpn I    BamH I
CTG CGC GAT GAT GAT GAT AAG GTA CCG GAT CCG
        Asp Asp Asp Asp Lys
```

The introduced adapter contains the cleavage sites for both Kpn I and BamH I as well as coding for the pentapeptide Asp-Asp-Asp-Asp-Lys, a peptidyl linkage specifically recognized by the endopeptidase enterokinase.

Using standard laboratory procedures, a 2.15 Kb Kpn I to BamH I fragment of plasmid pBenn#20, a fragment which spans the major portion of the ENV gene, was transferred onto plasmid pTrpLE-KAL via the same two restriction sites. The resultant plasmid, designated pTrpLE-KAL-ENV-#10, upon induction produced a reproducible pattern of protein products on SDS gels that were strongly reactive with the anti-AIDS sera (Figure 12).

The other specific clone was constructed so as to contain a 950 base pair fragment between the Pvu II and the Bgl II sites within the GAG gene of the AIDS virus. To construct this clone, a 1.1 Kb Sau3A fragment which encompasses the coding genes for the central region of the GAG open-reading frame was purified and cloned into the unique BamH I site of pSB8. We chose to use this particular vector in order to insert the Sau3A fragment in the "wrong" translational reading frame in reference

89821                                           25160-FF

to the LE protein. Our previous experience showed that attempts to insert this fragment in the "correct" reading frame would not result in proper translation. By using the "wrong" reading frame of pSB, many transformants containing the desired insertion were obtained. These were designated pSB8-GAG1.1. To regenerate the correct translational reading frame, pSB8-GAG1.1 DNA was cleaved with Sst II (within the LE) and Pvu II (about 130 bp into the Sau3A fragment). The 3' overhangs of the Sst II site were then removed using DNA polymerase I as described above, and the two blunt ends were recircularized using T4 ligase. Upon induction, one of the progeny transformants, pSB-Gag-#25, produced multiple polypeptides that were immuno-reactive with the anti-AIDS sera (Figure 12).

## EXAMPLE 13
### Analysis of AIDS Patient Sera with LE-AIDS Fusion Proteins

Western blotting analysis was used to demonstrate the usefulness of the different recombinant viral proteins produced in E. coli in detecting antibodies present in AIDS patients' sera. Serum samples were obtained random healthy blood donors, persons at high risk for contracting AIDS and patients suffering from lymphadenopathy, ARCS or AIDS. Extracts of the E. coli pSB8 vector alone, as a negative control, and of AIDS infected human lymphocytes, as a positive control, were electrophoresed and included in the assay of each serum. Figure 13 shows representative data from 19 serum samples.

A strong correlation between positive test results and AIDS, ARCS, and LAV infection was demonstrated.

Only a subset of the sera testing positive for antibodies to the AIDS virus and/or the ENV clone reacted with the GAG clones (pSB8-#45, pSB-Gag-#25 and pSB12-#31) or with the POL clones (pSB12-#67, pSB12-#68 and pSB12-#33).

The western blot analysis was performed as follows: E. coli extracts or AIDS virus-infected cell extracts (0.4 mg) were applied to SDS polyacrylamide gels (20 cm wide x 14 cm high x 0.15 cm) and electrophoresed according to the method of Laemmeli (Nature, 277, 680 (1970)). Western blot transfer was performed as described by Burnette (Anal. Bioch., 112, 195-203 (1981)). The blotted nitrocellulose (Schleicher & Schuell, BA83, 0.2 μm pore size) filters were then blocked, initially at 42°C for 30 min with TN buffer (10mM Tris-HCl, pH 7.4, and 155 mM NaCl) containing 5% non-fat dry milk, and subsequently at room temperature for 10 min with TN-TN buffer (10 mM Tris-HCl, pH 7.4, 155 mM NaCl, 0.3% Tween-20, 0.05% Nonidet P-40) containing 1% BSA. The nitrocellulose filters were then sliced longitudinally into strips of 1/4 inch in width. Serum samples were obtained from either healthy blood donors, individuals from "high-risk" groups, or patients with lymphadenopathy, ARC or AIDS. Serum from each individual was diluted 1000-fold in TN-TN-3% BSA and 8 ml were added to each separate chamber of a multi-trough plastic incubation tray. A set of nitrocellulose strips containing protein extracts derived from various E. coli clones or AIDS virus infected cells was placed into each chamber. Incubation was carried out at 4°C overnight with constant rocking. Upon removal of the sera, the strips were washed twice with 25 ml TN-TN buffer (5 minutes each) and twice with 25 ml TN-T buffer (TN-TN without NP-40). The filters were then incubated with 8 ml of diluted radioactively labelled protein A (200,000 dpm/ml of [$^{125}$I] protein A in TN-T-3% BSA). Incubation was carried out at room temperature for at least 45 minutes with constant rocking. The strips were then

-63-

washed three times with 25 ml of TN-TN plus 10mM EDTA
buffer followed by one wash with 25 ml of TN buffer
(5 minutes each wash). The washed nitrocellulose strips
were then air-dried and exposed to X-ray films.

### EXAMPLE 14

Expression of LE-ENV and LE-GAG

A starter culture of 50 ml LB culture media
containing 50 μg/ml ampicillin and 100 μg/ml
tryptophan was innoculated with E. coli cells containing
either the pTrpLE-KAL-ENV-#10 or the pSB8-Gag-#25
plasmid. The cultures were grown overnight, sonicated,
centrifuged, CNBr cleaved, and extracted with HOAc and
dialyzed as described in Example 4 above.

### EXAMPLE 15

Immunoreactivity of CNBr-Cleaved ENV Peptides

Western Blot analysis was used to determine the
immunoreactivities of the various CNBr polypeptide
fragments using anti-AIDS antibodies. Protein samples
from induced cultures containing pTrpLE-KAL-ENV-#10 or
pSB8-Gag-#25, with or without CNBr treatment, were
applied to SDS-polyacrylamide gels and electrophoresed.
The method of Burr and Burr (Meth. Enz., 96, 239-245) was
used for CNBr-treated samples, and the method of Laemmeli
(Nature, 277, 680 (1970)) was used for untreated
samples. Western blot transfer was performed as
described in Example 13.

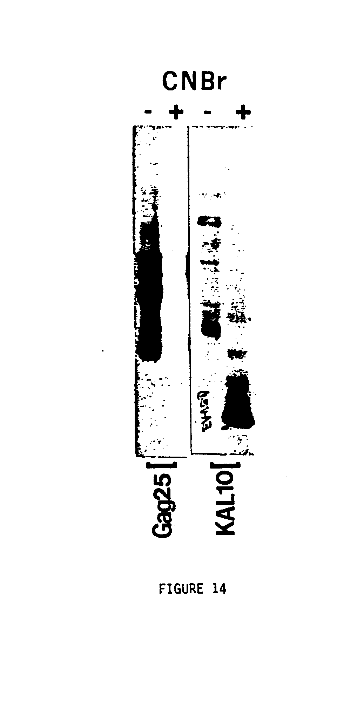

Western blot results indicated that while CNBr
treatment of LE-GAG fusion proteins generated by
pSB8-Gag-#25 completely destroyed any immunoreactive
epitope, similar chemical treatment of LE-ENV fusion
proteins produced by pTrpLE-KAL-ENV-#10 resulted in
peptides that retained strong immunoreactivity
(Figure 14). In fact, these peptides appear to react with anti-AIDS sera somewhat stronger immunologically than the intact fusion proteins.

## EXAMPLE 16
## Construction of High-Expression ENV Clone

The above results suggested that deletion mutants of Kal-10 which retain the coding sequences for important antigenic domains would be useful for diagnostic applications. Deletion of extraneous gene sequences, such as those that code for membrane binding domains, often result in progeny clones that are capable of synthesizing proteins of interest to a higher level.

Following this principle, we constructed a new plasmid (designated pENV-854ΔHB) as follows:

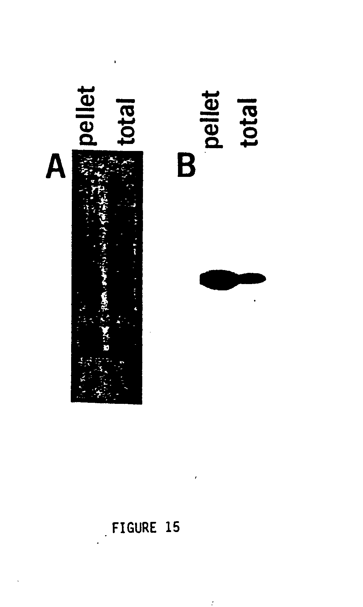

An 854 bp Bgl I I to BamH I fragment of pTrpLE-KAL-ENV-#10 was first cloned into the unique BamH I site of plasmid pSB12, yielding plasmid pSB12-854. Plasmid pSB12-854 DNA was further cleaved with Hind III and BamH I. The 5' overhangs were then filled using Klenow fragment and the resulting blunt ends recircularized using T4 ligase to produce the sequence AAGCTGATCC. The translational termination codon (TGA) generated by this process terminates the LE-ENV fusion protein at the Hind III-BamH I fusion site. The progeny transformant, pSB12-854ΔHB produced on induction a discrete 35 Kd polypeptide to more than 5% of the total cellular protein, as observed on an SDS-polyacrylamide gel stained with Coomasie Blue (Figure 15A). Western blot analysis of the total E. coli extract of pSB-854ΔHB verified that the 35 Kd protein was the only immunoreactive protein entity (Figure 15B).

The 35 Kd protein was subjected to CNBr cleavage, following the procedure set forth above. Western blot analysis demonstrated that the resulting polypeptide

-65-

fragments retain excellent immunoreactivity with anti-AIDS antibodies.

## EXAMPLE 17

### HPLC Fractionation of CNBr-ENV Peptide Fragments

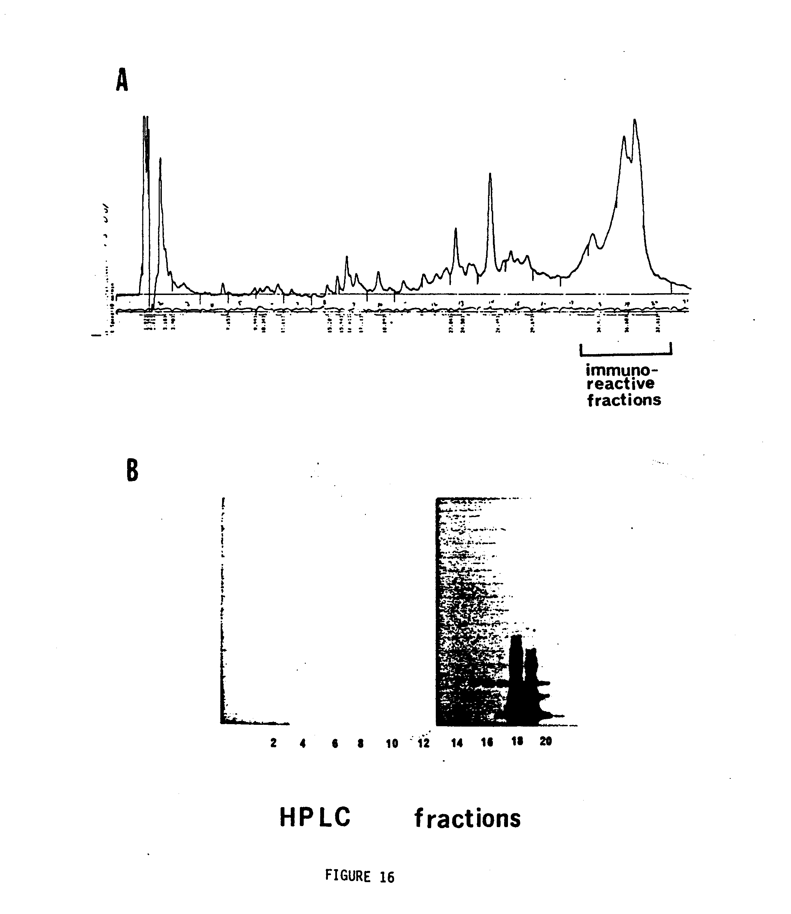

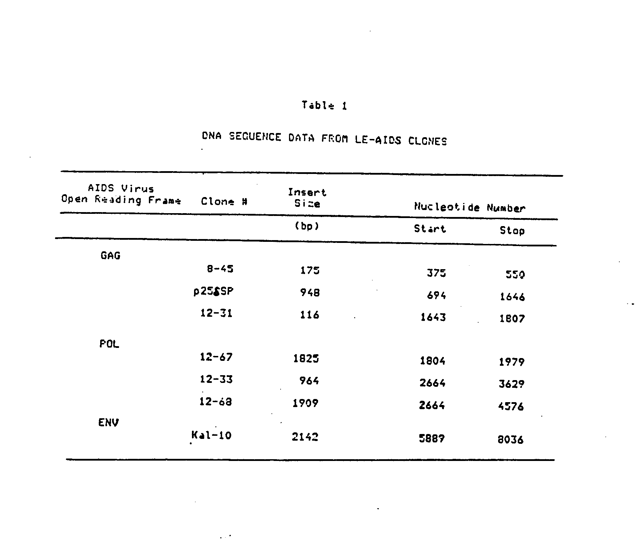

The CNBr-cleaved fragments obtained in Example 16 were applied to a 0.46 x 25 cm HPLC column packed with C18 silica beads (Vydac TP201, N-capped, 5 µM), which had been washed with 20% solvent B ($CH_3CN$ + 0.1% $CF_3COOH$). Solvent A was 0.1% aqueous $CF_3COOH$. The peptide fragments were eluted at a flow rate of 1.5 ml/min. with a gradient of 20% to 40% solvent B over a period of 30 min., followed by a gradient of 40% to 80% solvent B over an additional 20 min. The column fractions were collected and assayed immunologically by Western blot. The fractions which eluted between 34 and 40 minutes after injection contained immunoreactive ENV fragments (Figures 16A and 16B).

## EXAMPLE 18

### Western Blot With AIDS Patient Sera

Western Blot analysis was used to demonstrate the usefulness of the ENV-derived CNBr peptides in detecting anti-AIDS antibodies present in patient sera. Results indicated that, similar to the LE-ENV fusion proteins produced from pTrpLE-KAL-ENV-#10, pSB12-854, and pSB12-854ΔHB, CNBr fragments of SB12-854ΔHB served as ideal diagnostic markers for AIDS virus infection.

Claims

1.   A bacterial expression vector for the expression of fusion proteins, which vector comprises:
     a TrpLE DNA gene fragment;
     an AIDS virus DNA sequence;
     optionally a three-frame linker segment between said TrpLE fragment and said AIDS virus DNA sequence; and
     optionally a cleavable linker sequence between said TrpLE fragment and said AIDS virus DNA sequence.

2.   The vector of Claim 1 wherein said cleavable linker encodes Met, Asp-Pro, or Asp-Asp-Asp-Asp-Lys.

3.   The vector of Claim 1 wherein said AIDS virus DNA sequence is the ENV gene or a portion thereof, which preferably encodes the polypeptide KAL-10.

4.   The vector of Claim 3 which is pTrpLE-KAL, pTrpLE-KAL-ENV-#10, pSB12-854, or pSB12-854ΔHB.

5.   A method for producing a bacterial expression vector for the expression of fusion proteins, which method comprises:
     selecting a plasmid containing a TrpLE DNA gene;
     cleaving the TrpLE DNA gene with a restriction endonuclease; and
     inserting an AIDS virus DNA sequence.

6.   The method of Claim 5 which further comprises inserting a cleavable linker sequence before or after inserting said AIDS virus sequence.

7.    The method of Claim 6 wherein said cleavable
linker sequence encodes Met, Asp-Pro, or
Asp-Asp-Asp-Asp-Lys.

8.    A method for producing a bacterial expression
vector for the expression of fusion proteins, which
method comprises:
      selecting a plasmid containing a TrpLE DNA
      gene;
      cleaving the TrpLE DNA gene with a restriction
      endonuclease;
      inserting a three frame linker; and
      inserting an AIDS virus DNA sequence within
      the three frame linker.

9.    The method of Claim 8 wherein said AIDS virus
DNA sequence is KAL-10 or a portion thereof.

10.   The method of Claim 8 wherein said AIDS virus
DNA sequence comprises KAL-A, KAL-B, analogs or subsets
of KAL-A or KAL-B, or mixtures thereof, wherein KAL-A and
KAL-B have the following sequences:

Kal-A:
          Y Asp Asn Trp Arg Ser Glu Leu Tyr Lys
      Tyr Lys Val Val Lys Ile Glu Pro Leu Gly Val
      Ala Pro Thr Lys Ala Lys Arg Arg Val Val Gln
      Arg Glu Lys Arg Ala Val Gly Ile Gly Ala Leu
      Phe Leu Gly Phe Leu Gly Ala Ala Gly Ser Thr X;

Kal-B:
          Z Leu Thr Val Gln Ala Arg Gln Leu Leu
      Ser Gly Ile Val Gln Gln Gln Asn Asn Leu Leu
      Arg Ala Ile Glu Ala Gln Gln His Leu Leu Gln
      Leu Thr Val Trp Gly Ile Lys Gln Leu Gln Ala
      Arg Ile Leu Ala Val Glu Arg Tyr Leu Lys Asp
      Gln Gln Leu Leu Gly Ile Trp Gly Cys Ser Gly
      Lys Leu Ile Cys Thr Thr Ala Val Pro Trp Asn
      Ala Ser Trp Ser Asn Lys Ser Leu Glu Gln Ile
      Trp Asn Asn X.

wherein X is homoserine lactone or its hydrolysis or amidation products, methionine, or another naturally occurring amino acid, Y is Met-Arg or Arg and Z is Met-Thr or Thr.

11. The method of Claim 8 which further comprises inserting a cleavable linker sequence.

12. The method of Claim 8 wherein said cleavable linker sequence encodes Met, Asp-Pro, or Asp-Asp-Asp-Asp-Lys.

13. A fusion protein, which comprises:
   a TrpLE polypeptide fragment;
   an AIDS virus polypeptide, or a subset or analog thereof;
   optionally a cleavable linker oligopeptide between said TrpLE polypeptide fragment and said AIDS virus polypeptide; and
   optionally a three-frame linker oligopeptide segment between said TrpLE polypeptide fragment and said AIDS virus polypeptide.

14. The fusion protein of Claim 13 wherein said cleavable linker oligopeptide is Met, Asp-Pro, Asp-Asp-Asp-Asp-Lys.

15. The fusion protein of Claim 13 wherein said AIDS virus polypeptide is an ENV protein or a fragment thereof, preferably KAL-10.

-69-

16.    An AIDS polypeptide fragment, which comprises
the following sequence, its immunoreactive subsets and
analogs:

```
Y Asp Asn Trp Arg Ser Glu Leu Tyr Lys Tyr
Lys Val Val Lys Ile Glu Pro Leu Gly Val Ala
Pro Thr Lys Ala Lys Arg Arg Val Val Gln Arg
Glu Lys Arg Ala Val Gly Ile Gly Ala Leu Phe
Leu Gly Phe Leu Gly Ala Ala Gly Ser Thr X;
```

wherein X represents homoserine lactone or its
hydrolysis or amidation products, methionine, or another
natural or synthetic amino acyl residue and Y is Met-Arg
or Arg.

17.    An AIDS polypeptide fragment, which comprises
the following sequence, its immunoreactive subsets and
analogs:

```
Z Leu Thr Val Gln Ala Arg Gln Leu Leu Ser
Gly Ile Val Gln Gln Gln Asn Asn Leu Leu Arg
Ala Ile Glu Ala Gln Gln His Leu Leu Gln Leu
Thr Val Trp Gly Ile Lys Gln Leu Gln Ala Arg
Ile Leu Ala Val Glu Arg Tyr Leu Lys Asp Gln
Gln Leu Leu Gly Ile Trp Gly Cys Ser Gly Lys
Leu Ile Cys Thr Thr Ala Val Pro Trp Asn Ala
Ser Trp Ser Asn Lys Ser Leu Glu Gln Ile Trp
Asn Asn X;
```

where X represents homoserine lactone or its
hydrolysis or amidation products, methionine, or another
natural or synthetic amino acyl residue and Z is Met-Thr
or Thr.

89821                                          25160-FF

18. An immunoassay reagent for binding antibodies from humans exposed to AIDS virus, wherein said reagent is a fusion protein which comprises

a TrpLE polypeptide fragment;

an AIDS virus polypeptide; and

optionally a three-frame linker oligopeptide segment between said TrpLE oligopeptide fragment and said AIDS virus polypeptide.

19. The immunoassay reagent of Claim 18 wherein said AIDS virus polypeptide is an ENV protein or a fragment thereof, preferably KAL-10.

20. An immunoassay reagent for binding antibodies from humans exposed to AIDS virus, wherein said reagent is an AIDS polypeptide sequence, or an analog or subset thereof, which sequence comprises

```
Y   Asp Asn Trp Arg Ser Glu Leu Tyr Lys Tyr
Lys Val Val Lys Ile Glu Pro Leu Gly Val Ala
Pro Thr Lys Ala Lys Arg Arg Val Val Gln Arg
Glu Lys Arg Ala Val Gly Ile Gly Ala Leu Phe
Leu Gly Phe Leu Gly Ala Ala Gly Ser Thr X;
```

where X represents homoserine lactone or its hydrolysis or amidation products, methionine, or another natural or synthetic amino acyl residue and Y is Met-Arg or Arg.

21. An immunoassay reagent for binding antibodies from humans exposed to AIDS virus, wherein said reagent is an AIDS polypeptide sequence, or an analog or subset thereof, which sequence comprises

```
Z Leu Thr Val Gln Ala Arg Gln Leu Leu Ser
Gly Ile Val Gln Gln Gln Asn Asn Leu Leu Arg
Ala Ile Glu Ala Gln Gln His Leu Leu Gln Leu
Thr Val Trp Gly Ile Lys Gln Leu Gln Ala Arg
Ile Leu Ala Val Glu Arg Tyr Leu Lys Asp Gln
Gln Leu Leu Gly Ile Trp Gly Cys Ser Gly Lys
Leu Ile Cys Thr Thr Ala Val Pro Trp Asn Ala
Ser Trp Ser Asn Lys Ser Leu Glu Gln Ile Trp
Asn Asn X;
```

where X represents homoserine lactone or its hydrolysis or amidation products, methionine, or another natural or synthetic amino acyl residue and Z is Met-Thr or Thr.

22. An immunoassay reagent for binding antibodies from humans exposed to AIDS virus, wherein said reagent is a mixture of two or more AIDS polypeptide sequences, or analogs or subsets thereof, which sequences comprise

```
Y Asp Asn Trp Arg Ser Glu Leu Tyr Lys Tyr
Lys Val Val Lys Ile Glu Pro Leu Gly Val Ala
Pro Thr Lys Ala Lys Arg Arg Val Val Gln Arg
Glu Lys Arg Ala Val Gly Ile Gly Ala Leu Phe
Leu Gly Phe Leu Gly Ala Ala Gly Ser Thr X;
```

and

```
Z Leu Thr Val Gln Ala Arg Gln Leu Leu Ser
Gly Ile Val Gln Gln Gln Asn Asn Leu Leu Arg
Ala Ile Glu Ala Gln Gln His Leu Leu Gln Leu
Thr Val Trp Gly Ile Lys Gln Leu Gln Ala Arg
Ile Leu Ala Val Glu Arg Tyr Leu Lys Asp Gln
Gln Leu Leu Gly Ile Trp Gly Cys Ser Gly Lys
Leu Ile Cys Thr Thr Ala Val Pro Trp Asn Ala
Ser Trp Ser Asn Lys Ser Leu Glu Gln Ile Trp
Asn Asn X;
```

where X represents homoserine lactone or its hydrolysis or amidation products, methionine, or another natural or synthetic amino acyl residue, Y is Met-Arg or Arg and Z is Met-Thr or Thr.

23. The use of a fusion protein comprising:
a TrpLE polypeptide fragment;
an AIDS virus polypeptide;
optionally a cleavable linker oligopeptide between said TrpLE polypeptide fragment and said AIDS virus polypeptide; and
optionally a three-frame linker oligopeptide segment between said TrpLE polypeptide fragment and said AIDS virus polypeptide;
for the manufacture of a preparation inducing antibody formation in mammals.

24. The use of Claim 23 wherein said AIDS virus polypeptide is an ENV polypeptide or a fragment thereof, preferably KAL-10.

25. The use of Claim 23 or 24 wherein said antibodies are polyclonal or monoclonal and are useful for detecting the presence of AIDS virus.

26. The use of Claim 23 or 24 wherein said antibodies are neutralizing antibodies useful for conferring immunity to infection by AIDS virus.

27. The use of an AIDS polypeptide fragment comprising KAL-A, KAL-B, immunoreactive subsets or analogs of KAL-A or KAL-B or mixtures thereof, wherein KAL-A or KAL-B have the following sequences:

Kal-A:
```
      Y Asp Asn Trp Arg Ser Glu Leu Tyr Lys Tyr
Lys Val Val Lys Ile Glu Pro Leu Gly Val Ala
Pro Thr Lys Ala Lys Arg Arg Val Val Gln Arg
Glu Lys Arg Ala Val Gly Ile Gly Ala Leu Phe
Leu Gly Phe Leu Gly Ala Ala Gly Ser Thr X;
```

Kal-B:
```
      Z Leu Thr Val Gln Ala Arg Gln Leu Leu Ser
Gly Ile Val Gln Gln Gln Asn Asn Leu Leu Arg
Ala Ile Glu Ala Gln Gln His Leu Leu Gln Leu
Thr Val Trp Gly Ile Lys Gln Leu Gln Ala Arg
Ile Leu Ala Val Glu Arg Tyr Leu Lys Asp Gln
Gln Leu Leu Gly Ile Trp Gly Cys Ser Gly Lys
Leu Ile Cys Thr Thr Ala Val Pro Trp Asn Ala
Ser Trp Ser Asn Lys Ser Leu Glu Gln Ile Trp
Asn Asn X;
```

where X represents homoserine lactone, methionine, or another natural or synthetic amino acyl residue, Y is Met-Arg or Arg and Z is Met-Thr or Thr, for the manufacture of a preparation inducing antibody formation in mammals.

28. The use of an AIDS polypeptide fragment comprising KAL-A, KAL-B, immunoreactive subsets or analogs of KAL-A or KAL-B, or mixtures thereof, wherein KAL-A and KAL-B have the following sequences:

Kal-A:

```
Y Asp Asn Trp Arg Ser Glu Leu Tyr Lys Tyr
  Lys Val Val Lys Ile Glu Pro Leu Gly Val Ala
  Pro Thr Lys Ala Lys Arg Arg Val Val Gln Arg
  Glu Lys Arg Ala Val Gly Ile Gly Ala Leu Phe
  Leu Gly Phe Leu Gly Ala Ala Gly Ser Thr X;
```

Kal-B:

```
Z Leu Thr Val Gln Ala Arg Gln Leu Leu Ser
  Gly Ile Val Gln Gln Gln Asn Asn Leu Leu Arg
  Ala Ile Glu Ala Gln Gln His Leu Leu Gln Leu
  Thr Val Trp Gly Ile Lys Gln Leu Gln Ala Arg
  Ile Leu Ala Val Glu Arg Tyr Leu Lys Asp Gln
  Gln Leu Leu Gly Ile Trp Gly Cys Ser Gly Lys
  Leu Ile Cys Thr Thr Ala Val Pro Trp Asn Ala
  Ser Trp Ser Asn Lys Ser Leu Glu Gln Ile Trp
  Asn Asn X;
```

where X represents homoserine lactone, methionine, or another natural or synthetic amino acyl residue, Y is Met-Arg or Arg and Z is Met-Thr or Thr for the manufacture of a medicament for reducing or preventing AIDS virus infection in a mammal by competitively binding to cellular viral receptors.

29. The use of a fusion protein comprising
a TrpLE polypeptide fragment; and
an AIDS virus polypeptide recognized by a mammalian cell virus receptor
for the manufacture of a medicament for reducing or preventing AIDS virus infection in a mammal by competitively binding to cellular viral receptors.

30. A vaccine which comprises a fusion protein according to any one of Claims 13 to 15 or a portion thereof and a pharmaceutically acceptable excipient, preferably an adjuvant.

-75-

31.    A medicament for reducing or preventing AIDS virus infection in a mammal by competitively binding to cellular viral receptors, which comprises a fusion protein comprising

a TrpLE polypeptide fragment;  and
an AIDS virus polypeptide recognized by a mammalian cell virus receptor.

FIGURE 1

FIGURE 2

mRNA from Plasmid pTrp-Sst II-HuGRF

```
                                                              50
AAGTTCACGT AAAAAGGGTA TCGACA ATG AAA GCA ATT TTC GTA CTG AAA GGT TCA CTG GAC
                            Met Lys Ala Ile Phe Val Leu Lys Gly Ser Leu Asp

                                                         100
AGA GAT CTC GAC AGC CGT ATT GAA CTG GAA ATG CGT ACC GAT CAT AAA GAG CTG TCT GAA
Arg Asp Leu Asp Ser Arg Ile Glu Leu Glu Met Arg Thr Asp His Lys Glu Leu Ser Glu

                              150
CAT CTG ATG CTG GTT GAT CTC GCC CGT AAT GAT CTG GCA CSC ATT TGC ACC CCC GGC AGC
His Leu Met Leu Val Asp Leu Ala Arg Asn Asp Leu Ala Arg Ile Cys Thr Pro Gly Ser

              200
CGC TAC GTC GCC GAT CTC ACC AAA GTT GAC CGT TAT TCC TAT GTG ATG CAC CTC GTC TCT
Arg Tyr Val Ala Asp Leu Thr Lys Val Asp Arg Tyr Ser Tyr Val Met His Leu Val Ser

    250                                                                  300
CGC GTA GTC GGC GAA CTG CGT CAC GAT CTT GAC GCC CTG CAC GCT TAT CGC GCC TGT ATG
Arg Val Val Gly Glu Leu Arg His Asp Leu Asp Ala Leu His Ala Tyr Arg Ala Cys Met

                                                    350
AAT ATG GGG ACG TTA AGC GGT GCG CCG AAA GTA CGC GCT ATG CAG TTA ATT GCC GAG GCG
Asn Met Gly Thr Leu Ser Gly Ala Pro Lys Val Arg Ala Met Gln Leu Ile Ala Glu Ala

              Sst II                              400
GAA GGT CGT CGC CGC GGG ATG GTC CAT ATG TAC GCT GAT GCT ATA TTC ACT AAC TCT TAC
Glu Gly Arg Arg Arg Gly Met Val His Met TYR ALA ASP ALA ILE PHE THR ASN SER TYR

                                450
CGC AAG GTT CTG GGC CAA TTA TCA GCA CGC AAG CTT CTG CAG GAC ATT CTT AGT CGA CAA
ARG LYS VAL LEU GLY GLN LEU SER ALA ARG LYS LEU LEU GLN ASP ILE LEU SER ARG GLN

                  500
CAG GGC GAA TCT AAC CAG GAG CGT GGC GCC AGA GCC AGA CTG TGA TCC GTCGGATGTC
GLN GLY GLU SER ASN GLN GLU ARG GLY ALA ARG ALA ARG LEU ---

    550                                        600
CAAAAGGTCG CCACTTTTCC ACTTCCTGTT TCATTCTCGT TCCTTAAGAA AGTTACACAG GAATAGTATC

                                650
GAATCCTTGC CGAGTTTGAC TCAATTAGTT CAGTCAGTTT CAGGATATTA GTCATCTCTA CATTGATTAT

        700                                                    750
GAGTATTCAG AAATTCCTTA AATATTCTGA CAAATGCTCT TTCCCTAAAC TCCCCCCATA AAAAAACCCG

    -760
CCGAAGCGGG TTTTT
```

FIG. 3A

mRNA from Plasmid pTrp-BssH II-HuGRF

```
                                                          50
                                          .    .         .    .         .
AAGTTCACGT AAAAAGGGTA TCGACA ATG AAA GCA ATT TTC GTA CTG AAA GGT TCA CTG GAC
                            Met Lys Ala Ile Phe Val Leu Lys Gly Ser Leu Asp

                                                    100
    .         .         .         .         .         .         .
AGA GAT CTC GAC AGC CGT ATT GAA CTG GAA ATG CGT ACC GAT CAT AAA GAG CTG TCT GAA
Arg Asp Leu Asp Ser Arg Ile Glu Leu Glu Met Arg Thr Asp His Lys Glu Leu Ser Glu

                              150
    .         .         .         .         .         .         .
CAT CTG ATG CTG GTT GAT CTC GCC CGT AAT GAT CTG GCA CGC ATT TGC ACC CCC GGC AGC
His Leu Met Leu Val Asp Leu Ala Arg Asn Aso Leu Ala Arg Ile Cys Thr Pro Gly Ser

                   200
    .         .         .         .         .         .         .
CGC TAC GTC GCC GAT CTC ACC AAA GTT GAC CGT TAT TCC TAT GTG ATG CAC CTC GTC TCT
Arg Tyr Val Ala Asp Leu Thr Lys Val Asp Arg Tyr Ser Tyr Val Met His Leu Val Ser

      250                                                                    300
    .         .         .         .         .         .         .
CGC GTA GTC GGC GAA CTG CGT CAC GAT CTT GAC GCC CTG CAC GCT TAT CGC GCC TGT ATG
Arg Val Val Gly Glu Leu Arg His Asp Leu Asp Ala Leu His Ala Tyr Arg Ala Cys Met

                                                     350
    .         .         .         .         .         .         .
AAT ATG GGG ACG TTA AGC GGT GCG CCG AAA GTA CGC GCT ATG CAG TTA ATT GCC GAG GCG
Asn Met Gly Thr Leu Ser Gly Ala Pro Lys Val Arg Ala Met Gln Leu Ile Ala Glu Ala

                                           400
    .         .         .         .         .         .         .
GAA GGT CGT CGC CGC GGC AGC TAC GGC GGC GCG GTA GGT TAT TTC ACC GCG CAT GGC GAT
Glu Gly Arg Arg Arg Gly Ser Tyr Gly Gly Ala Val Gly Tyr Phe Thr Ala His Gly Asp

                                  450
    .         .         .         .         .         .         .
CTC GAC ACC TGC ATT GTG ATC CGC TCG GCG CTG GTG GAA AAC GGT ATC GCC ACC GTG CAA
Leu Asp Thr Cys Ile Val Ile Arg Ser Ala Leu Val Glu Asn Gly Ile Ala Thr Val Gln

               500
    .         .         .         .         .         .         .
GCG GGT GCT GGT GTA GTC CTT GAT TCT GTT CCG CAG TCG GAA GCC GAC GAA ACC CGT AAC
Ala Gly Ala Gly Val Val Leu Asp Ser Val Pro Gln Ser Glu Ala Asp Glu Thr Arg Asn

         550              .BssH II                                        600
    .         .         .         .         .         .         .
AAA GCC CGC GCT GTA CTG CGC GCT ATG TAC GCT GAT GCT ATA TTC ACT AAC TCT TAC CGC
Lys Ala Arg Ala Val Leu Arg Ala Met TYR ALA ASP ALA ILE PHE THR ASN SER TYR ARG

                                                     650
    .         .         .         .         .         .         .
AAG GTT CTG GGC CAA TTA TCA GCA CGC AAG CTT CTG CAG GAC ATT CTT AGT CGA CAA CAG
LYS VAL LEU GLY GLN LEU SER ALA ARG LYS LEU LEU GLN ASP ILE LEU SER ARG GLN GLN

                                  700
    .         .         .         .         .         .
GGC GAA TCT AAC CAG GAG CGT GGC GCC AGA GCC AGA CTG TGA TCCGTC GGATGTCCAA
GLY GLU SER ASN GLN GLU ARG GLY ALA ARG ALA ARG LEU ---

                   750        —
    .         .         .         .         .         .
AAGGTCGCCA CTTTTCCACT TCCTGTTTCA TTCTCGTTCC TTAAGAAAGT TACACAGGAA TAGTATCGAA

      800                                                     850
    .         .         .         .         .
TCCTTGCCGA GTTTGACTCA ATTAGTTCAG TCAGTTTCAG GATATTAGTC ATCTCTACAT TGATTATGAG

                                  900
    .         .         .         .         .         .
TATTCAGAAA TTCCTTAAAT ATTCTGACAA ATGCTCTTTC CCTAAACTCC CCCCATAAAA AAACCCGCCG

      940
AAGCGGGTTT TT
```

FIG. 3B

Purification of HGRF$_{Leu27}$ from E. coli Cells
Producing a TrpLE-HGRF Fusion Protein

```
                    CELLS
                      │   Octonol, N2 gas
                      │   Sonicate
                      │
                CELL SONICATE
                      │   Centrifuge
                      ├──────────────────────┐
             LOW SPEED PELLET                 │
                      │                    CYTOSOL
                      │   70% Formic Acid
                      │   Octonol, N2 gas
                      │
              PRE-CNBr SOLUTION
                      │   CNBr, 6hr
                      │   Rotory Evaporate
                      │   Lyophilize
                      │   Extract with 1N HAc
                      │   Centrifuge
                      │
                HAc EXTRACT
                      │
                    PELLET
                      │   Dialize
                      │   Centrifuge
                      ├──────────────────────┐
                 DIALYSATE                    │
                      │                     PELLET
              CM-CELLULOSE POOL
                      │
                  HPLC POOL
                      │
                  G-50 POOL
                      │   Lyophilize 3-4 times
        FINAL PRODUCT - HuGRF$_{Leu27}$
```

FIGURE 4

0212532

6/18

FIGURE 5

FIGURE 6

FIGURE 7

**A.** TrpLE (BssH II)GRF

**B.** TrpLE (Sac II)GRF

FIGURE 8

A.            Stained Gel

B.            Western Blot

FIGURE 9

FIGURE 10

12/18

FIGURE 11

13/18

FIGURE 12

FIGURE 13

FIGURE 14

0212532

FIGURE 15

0212532

17/8

A

immuno-
reactive
fractions

B

2  4  6  8  10  12  14  16  18  20

HPLC      fractions

FIGURE 16

१४/१५

Table 1

DNA SEGUENCE DATA FROM LE-AIDS CLONES

| AIDS Virus Open Reading Frame | Clone # | Insert Size (bp) | Nucleotide Number Start | Stop |
|---|---|---|---|---|
| GAG | | | | |
| | 8-45 | 175 | 375 | 550 |
| | p25SSP | 948 | 694 | 1646 |
| | 12-31 | 116 | 1643 | 1807 |
| POL | | | | |
| | 12-67 | 1825 | 1804 | 1979 |
| | 12-33 | 964 | 2664 | 3629 |
| | 12-68 | 1909 | 2664 | 4576 |
| ENV | | | | |
| | Kal-10 | 2142 | 5887 | 8036 |

## EUROPEAN SEARCH REPORT

**European Patent Office**

Application number

### DOCUMENTS CONSIDERED TO BE RELEVANT

EP 86111099.7

| Category | Citation of document with indication, where appropriate, of relevant passages | Relevant to claim | CLASSIFICATION OF THE APPLICATION (Int. Cl.4) |
|---|---|---|---|
| A | <u>EP - A2 - 0 139 216</u> (KYOWA HAKKO KOGYO CO., LTD)<br><br>* Claims 4,5 *<br><br>-- | 1,13 | C 12 N 15/00<br>C 07 K 13/00<br>C 07 K 15/00<br>C 07 K 7/06<br>G 01 N 33/53<br>G 01 N 33/68<br>A 61 K 39/12<br>A 61 K 37/02 |
| D,A | GENE, vol. 21, no. 1,2, January/ February 1983 (Amsterdam)<br><br>A.R. DAVIS et al. "Immune response to human influenza virus hemag- glutinin expressed in Escherichia coli"<br>pages 273-284<br><br>* Summary *<br><br>-- | 1,13 | |
| D,A | <u>GB - A - 2 073 203</u> (GENENTECH, INC.)<br><br>* Claims 1-6,8,11-16,18 *<br><br>-- | 1 | |
| D,A | JOURNAL OF BACTERIOLOGY, vol. 133, no. 1, January 1978 (Washington DC)<br><br>G.F. MIOZZARI et al. "Translation of the Leader Region of the Escherichia coli Tryptophan Operon"<br>pages 1457-1466<br><br>* Totality *<br><br>-- | 1 | **TECHNICAL FIELDS SEARCHED (Int. Cl.4)**<br><br>C 12 N<br>C 07 K<br>G 01 N<br>A 61 K |
| P,A | <u>EP - A1 - 0 176 341</u> (ELI LILLY AND COMPANY)<br><br>* Claims 1-3 *<br><br>-- | 1 | |

The present search report has been drawn up for all claims

| Place of search | Date of completion of the search | Examiner |
|---|---|---|
| VIENNA | 14-11-1986 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503. 03.82

European Patent Office

**EUROPEAN SEARCH REPORT**

| DOCUMENTS CONSIDERED TO BE RELEVANT | EP 86111099.7 |
|---|---|

| Category | Citation of document with indication, where appropriate, of relevant passages | Relevant to claim | CLASSIFICATION OF THE APPLICATION (Int. Cl.4) |
|---|---|---|---|
| D,A | NATURE, vol. 313, January 24, 1985 (New York, London) <br><br> L.RATNER et al. "Complete nucleotide sequence of the AIDS virus, HTLV-III" <br> pages 277-284 <br><br>      * Totality * <br><br> ---- | 1 | |

TECHNICAL FIELDS SEARCHED (Int. Cl.4)

The present search report has been drawn up for all claims

| Place of search | Date of completion of the search | Examiner |
|---|---|---|
| VIENNA | 14-11-1986 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82